# EUROPEAN PATENT SPECIFICATION

(11) **EP 4 346 857 B1**
(45) Date of publication and mention of the grant of the patent: **19.08.2026**
(21) Application number: 22731145.3
(22) Date of filing: 25.05.2022
(51) Int. Cl.: A61K 35/74, A61K 31/047, A61K 31/155, A61K 33/24, A61P 3/10

(54) **INSULIN-SENSITIZING AGENT AND BUTYRATE-PRODUCING BACTERIUM**
INSULINSENSIBILISIERENDES MITTEL UND BUTYRATPRODUZIERENDES BAKTERIUM
AGENT INSULINOSENSIBILISANT ET BACTÉRIE PRODUCTRICE DE BUTYRATE

(30) Priority: 28.05.2021 NL 2028324
(43) Date of publication of application: 10.04.2024
(73) Proprietor: Caelus Lifesciences IP B.V., 2629 JA Delft (NL)
(72) Inventor: DE VOS, Willem Meindert, 1082 PB AMSTERDAM (NL); STERKMAN, Lucas Gerardus Willibrordus, 1852 RM HEILOO (NL); BUI, Thi Phuong Nam, 6721 ZE BENNEKOM (NL); GÜL, Ismail Sahin, 2200 HERENTALS (BE)
(74) Representative: EP&C
(86) International application number: PCT/EP2022/064277
(87) International publication number: WO 2022/248588

(56) References cited:
- WO-A1-2016/110585
- WO-A1-2021/028585
- MANIAR KUNAL ET AL: "A story of metformin-butyrate synergism to control various pathological conditions as a consequence of gut microbiome modification: Genesis of a wonder drug?", PHARMACOLOGICAL RESEARCH, ELSEVIER, AMSTERDAM, NL, vol. 117, 8 December 2016 (2016-12-08), pages 103 - 128, XP029921250, ISSN: 1043-6618, DOI: 10.1016/J.PHRS.2016.12.003
- GILIJAMSE PIM W. ET AL: "Treatment with Anaerobutyricum soehngenii: a pilot study of safety and dose-response effects on glucose metabolism in human subjects with metabolic syndrome", vol. 6, no. 1, 1 December 2020 (2020-12-01), XP055879874, Retrieved from the Internet <URL:https://www.nature.com/articles/s41522-020-0127-0.pdf> DOI: 10.1038/s41522-020-0127-0
- ALI ATHER ET AL: "Chromium Picolinate for the Prevention of Type 2 Diabetes", TREATMENT STRATEGIES. DIABETES, 1 January 2011 (2011-01-01), England, pages 34 - 40, XP055879906, Retrieved from the Internet <URL:https://www.ncbi.nlm.nih.gov/pmc/articles/PMC4169208/pdf/nihms308458.pdf> [retrieved on 20220117]
- WATKINS OLIVER C. ET AL: "A review of the role of inositols in conditions of insulin dysregulation and in uncomplicated and pathological pregnancy", CRITICAL REVIEWS IN FOOD SCIENCE AND NUTRITION, 7 December 2020 (2020-12-07), USA, pages 1 - 49, XP055879915, ISSN: 1040-8398, DOI: 10.1080/10408398.2020.1845604
- CHUKWUMA CHIKA IFEANYI ET AL: "Sorbitol increases muscle glucose uptake ex vivo and inhibits intestinal glucose absorption ex vivo and in normal and type 2 diabetic rats", vol. 42, no. 4, 1 April 2017 (2017-04-01), CA, pages 377 - 383, XP055880036, ISSN: 1715-5312, Retrieved from the Internet <URL:http://dx.doi.org/10.1139/apnm-2016-0433> DOI: 10.1139/apnm-2016-0433
- LIU YILUN ET AL: "Intestinal Bacteria Encapsulated by Biomaterials Enhance Immunotherapy", FRONTIERS IN IMMUNOLOGY, vol. 11, 10 February 2021 (2021-02-10), XP055880283, DOI: 10.3389/fimmu.2020.620170

## Description

### TECHNICAL FIELD

The present invention relates to the field of preventing and/or treating insulin resistance and insulin resistance-related conditions chosen from type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

### BACKGROUND OF THE DISCLOSURE

Insulin resistance is a medical condition wherein cells are resistant to the insulin, leading to hyperglycaemia, or high blood sugar level. Long-term complications of hyperglycaemia include cardiovascular disease, nerve damage, kidney damage/failure, damage to the blood vessels, and potentially blindness.

Insulin resistance may be treated or prevented by improving the insulin sensitivity in subjects. Several compounds are known to improve the insulin sensitivity. Common insulin-sensitizing dietary supplements include, among others, chromium, inositol, berberin, and magnesium. Common insulin-sensitizing medicinal products include, among others, glibenclamide, pioglitazone, rosiglitazone, and biguanide. As an example of the biguanides, the drug metformin may be used as an insulin-sensitizing medicinal product and is a first-line medication for the treatment of type 2 diabetes mellitus.

Insulin-sensitizing agents may improve the sensitivity of peripheral tissues to insulin, which results in decreased circulating insulin levels. However, the molecular mechanisms involved in alleviating insulin resistance by insulin-sensitizing compounds are often poorly understood.

Maniar Kunal et al (Pharmacological Research, Elsevier, Amsterdam, NL, part 117, 8 December 2016, pages 103-128) discloses that metformin results in a gradual increase in butyrate-producing taxa in the gut in patients with NAFLD, PCOS, type 2 diabetes.

There is a need in the art for new interventions that improve the therapeutic effect of insulin-sensitizing agents. In particular, the therapeutic effect of insulin-sensitizing dietary supplements to enhance insulin sensitivity and prevent/treat metabolic disease, remains questionable (Ali et al. Treat Strategies Diabetes. 2011; 3(1): 34-40). In comparison, insulin-sensitizing medicinal products such as metformin are often more clinically effective in preventing/treating metabolic diseases, including insulin resistance and insulin resistance-related conditions. Nevertheless, there is a need for new strategies to reduce the effective dose of insulin-sensitizing medicinal products, and/or further increase its efficacy, as an approach for managing and reducing adverse events (Bruckbauer et al. Diabetes Metab Syndr Obes. 2013; 6: 93-102).

It is an object of the present disclosure, amongst other objects, to address the above need in the art to provide a new and/or improved strategy for preventing and/or treating metabolic diseases, such as insulin resistance and insulin resistance-related conditions, preferably chosen from type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

Moreover, it is an object of the present disclosure, among other objects, to improve the therapeutic effects of insulin-sensitizing agents.

### SUMMARY OF THE DISCLOSURE

The invention is as set out in the appended claims.

The present inventors surprisingly found that the treatment of a subject with both an insulin-sensitizing agent and a butyrate-producing bacterium according to the present disclosure may provide a synergistic therapeutic effect in the prevention or treatment of metabolic diseases, insulin resistance, and insulin resistance-related conditions.

The present disclosure teaches a combination comprising at least one insulin-sensitizing agent and at least one butyrate-producing bacterium to provide a synergistic therapeutic effect in the prevention or treatment of metabolic diseases, insulin resistance, and insulin resistance-related conditions.

As part of the combination in the current disclosure, the insulin-sensitizing agent may be administered (e.g. to a human subject) separately, sequentially or simultaneously to the butyrate-producing bacterium, wherein the insulin-sensitizing agent and the butyrate-producing bacterium may be in separate formulations. Alternatively, the insulin-sensitizing agent may be administered (e.g. to a human subject) together with the butyrate-producing bacterium in a single composition.

The butyrate-producing bacterium as taught herein is preferably chosen from *Anaerobutyricum species*/*Eubacterium* species, *Intestinimonas* species and/or *Anaerostipes* species. The butyrate-producing bacterium preferably is *Eubacterium hallii, Anaerobutyricum soehngenii, Anaerobutyricum hallii, Intestinimonas butyriciproducens,* and/or *Anaerostipes rhamnosivorans.*

The present disclosure provides a use, among other objects, to improve the beneficial therapeutic effects of insulin-sensitizing agents by combining such insulin-sensitizing agent with at least one butyrate-producing bacterium.

The present disclosure furthermore provides, among other objects, a new and improved strategy for preventing and/or treating metabolic disease, insulin resistance and insulin resistance-related conditions, preferably chosen from type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

### DETAILED DESCRIPTION OF THE DISCLOSURE

The invention is as set out in the appended claims.

The present disclosure relates to the use of at least one insulin-sensitizing agent, preferably chosen from the group consisting of
- chromium;
- inositol;
- metformin; and/or
- sorbitol

for e.g. preventing and/or treating metabolic disease, insulin resistance and/or insulin resistance-related conditions,
wherein the insulin-sensitizing agent can be combined with a butyrate-producing bacterium preferably chosen from the group consisting of
   - *Anaerobutyricum soehngenii or Anaerobutyricum hallii,* or relative thereof having a 16S rRNA gene sequence with at least 97% sequence identity with SEQ ID NO:1 or SEQ ID NO:2;
   - *Intestinimonas butyriciproducens* or relative thereof having a 16S rRNA gene sequence with at least 97% sequence identity with SEQ ID NO:3; and/or
   - *Anaerostipes rhamnosivorans* or relative thereof having a 16S rRNA gene sequence with at least 97% sequence identity with SEQ ID NO:4.

Of note all these butyrate-producing bacteria can produce butyrate from sugars and acetate but typically also are capable of the unusual conversion of producing butyrate from lactate and acetate. This is of specific importance as acetate and the undesired lactate are produced in the small intestine and this is also the site where the insulin-sensitizing agents can have their effect.

### COMBINATION FOR USE IN PREVENTION AND/OR TREATMENT OF METABOLIC DISEASES

The combination according to the present disclosure, comprising at least one insulin-sensitizing agent and at least one butyrate-producing bacterium, finds a particular use in the treatment and/or prevention of metabolic disease, such as insulin resistance and insulin resistance-related conditions, preferably chosen from type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH), most preferably insulin resistance, type 1 diabetes mellitus, type 2 diabetes mellitus, and metabolic syndrome.

The term 'metabolic disease', or metabolic disorder, relates to conditions that disrupt normal metabolism, the process of converting food to energy on a cellular level. Metabolic disease can be congenital or acquired. Insulin resistance, metabolic syndrome, type 1 diabetes mellitus, and type 2 diabetes mellitus are common examples of metabolic diseases.

The term 'insulin resistance' is well known by the skilled person. Within the present disclosure, the term encompasses all conditions diagnosed as 'insulin resistance' by an (authorized) medical practitioner. The term 'insulin resistance' as used herein preferably refers to peripheral insulin resistance and/or hepatic insulin resistance. Insulin resistance may for example be diagnosed by the gold standard which is the "hyperinsulinemic euglycemic clamp" (DeFronzo RA, Tobin JD, Andres R, Am J Physiol. 1979 Sep;237(3):E214-23). This method measures the amount of glucose necessary to compensate for an increased insulin level without causing hypoglycemia. The procedure may take two hours and typically involves the following steps: Through a peripheral vein, insulin is infused at 10-120 mU per m² per minute. In order to compensate for the insulin infusion, glucose 20% is infused to maintain blood sugar levels between 5 and 5.5 mmol/L. The rate of glucose infusion is determined by checking the blood sugar levels every five to ten minutes (Muniyappa R, Lee S, Chen H, Quon MJ, January 2008, American Journal of Physiology. Endocrinology and Metabolism. 294 (1): E15-26). The rate of glucose infusion during the last thirty minutes of the test determines insulin sensitivity. If high levels (7.5 mg/min or higher) are required, the patient is insulin sensitive. Low levels (4.0 mg/min or lower) indicate insulin resistance. Levels between 4.0 and 7.5 mg/min are not definitive, and suggest "impaired glucose tolerance," an early sign of insulin resistance. Other methods of assessing insulin sensitivity or resistance include the "insulin-suppression test" (IST), "minimal model analysis of frequently sampled intravenous glucose tolerance test" (FSIVGTT), or the "oral glucose tolerance test" (OGTT).The homeostatic model assessment for insulin resistance (HOMA or HOMA-IR) is an alternative method used to determine and quantify insulin resistance in a fasting steady-state condition, and correlates reasonably with the golden standard (See e.g. Matthews DR, Hosker JP, Rudenski AS, Naylor BA, Treacher DF, Turner RC (1985). Diabetologia. 28 (7): 412-9. doi:10.1007/BF00280883. PMID 3899825; and/or A. S. Rudenski; D. R. Matthews; J. C. Levy; R. C. Turner (1991) Metabolism. 40 (9): 908-917). A HOMA(-IR) score that deviates from a reference range can indicate insulin resistance. HOMA(-IR) has been widely applied in epidemiological studies and in experimental research. HOMA(-IR) denotes a value which represents an estimation for insulin resistance, derived from dividing Insulin and Glucose levels in the blood of a person. The HOMA(-IR) value can be calculated by the following equation: $H = \frac{Glucose \times Insulin}{405}$ wherein H is the HOMA(-IR) value expressed in mg/dL, Glucose represents fasting glucose levels in the blood expressed in mmol/L, Insulin represents fasting insulin levels in the blood expressed in mIU/L. IU (relating to enzyme activity) is an abbreviation of International Units, also called enzyme units. The skilled person is familiar with the methods used to quantify these levels. Enzyme activity is the amount of substrate converted per unit of time. One IU equals the conversion of one µmol of substrate per minute. For an individual with normal insulin sensitivity, HOMA(-IR) may equal 1. The upper limit of normal HOMA(-IR) is frequently considered to be 2.0, although the normal HOMA(-IR) may be dependent on the characteristics of the population subgroup. Typically, a healthy subject has a HOMA-IR value below 2.0 mg/dL, preferably a HOMA-IR value below 1.9, 1.8, or 1.7 mg/dL, more preferably a HOMA-IR value below 1.6, 1.5, 1.4, 1.3, 1.2, 1.1 mg/dL, and most preferred a HOMA-IR value below 1.0. A fasting serum insulin level greater than 25 mU/L or 174 pmol/L has also been considered as indicating insulin resistance.

The term 'insulin resistance-related conditions' may refer to conditions that are caused and/or exaggerated by insulin resistance. Alternatively, 'insulin resistance-related conditions' may refer to conditions wherein the symptoms have been associated with insulin resistance.

Prevention/and or treatment of insulin resistance in insulin resistance-related conditions may relieve symptoms, mitigate progression of disease, and/or reverse disease. Prevention/and or treatment of insulin resistance in these conditions may also mitigate the chance of acquiring additional co-morbidities associated with the insulin resistance-related condition. Insulin resistance-related conditions disclosed herein are type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH), preferably insulin resistance, type 1 diabetes, type 2 diabetes, and metabolic syndrome.

The term 'type 1 diabetes mellitus' is well-known to the skilled person. Type 1 diabetes mellitus is thought to result from an autoimmune destruction of pancreatic β-cells, and the predominant pathophysiology may be an almost absolute insulinopenia. Insulin resistance has also been described in type 1 diabetes mellitus and may be a potential target for intervention in addition to insulin therapy. Insulin resistance is often observed in subjects suffering type 1 diabetes mellitus during pubertal development and inter-current illness.

The term 'type 2 diabetes mellitus' is well-known to the skilled person. Patients with type 2 diabetes mellitus are characterized by high blood sugar, insulin resistance, and relative lack of insulin. These patients usually have passed through an earlier stage of insulin resistance, although this earlier stage often goes undiagnosed. It is widely accepted that insulin resistance is a powerful predictor of future development of type 2 diabetes mellitus, and also is the main therapeutic target once high blood sugar is present.

The term 'dyslipidemia' is well-known to the skilled person. Dyslipidemia is characterized by abnormal amount of lipids in the blood. In developed countries, most dyslipidemias are hyperlipidemias, or an elevation of lipids in the blood, which has a strong relation to cardiovascular disease. Insulin resistance, and the compensatory hyperinsulinemia that results, has been linked to dyslipidemia. The characteristic dyslipidemia of insulin resistance consists of elevated triglyceride and triglyceride-rich lipoprotein levels, low levels of high-density lipoprotein cholesterol, and increased concentrations of small, dense low-density lipoprotein cholesterol.

The term 'metabolic syndrome' is well-known by the skilled person. Within the present disclosure, the term encompasses all conditions diagnosed as "metabolic syndrome" by an (authorized) medical practitioner. For example, metabolic syndrome may be diagnosed if a patient has at least two, or at least three of the following traits:
- Large waist - A waistline that measures at least 35 inches (89 centimeters) for women and 40 inches (102 centimeters) for men;
- High triglyceride level - 150 milligrams per deciliter (mg/dL), or 1.7 millimoles per liter (mmol/L), or higher of this type of fat found in blood;
- Reduced "good" or HDL cholesterol - Less than 40 mg/dL (1.04 mmol/L) in men or less than 50 mg/dL (1.3 mmol/L) in women of high-density lipoprotein (HDL) cholesterol;
- Increased blood pressure - 130/85 millimeters of mercury (mm Hg) or higher;
- Elevated fasting blood sugar - 100 mg/dL (5.6 mmol/L) or higher.

Metabolic syndrome is an example of an insulin resistance-related condition. Most people who have metabolic syndrome have insulin resistance. Insulin resistance has been suggested as an important underlying cause of metabolic syndrome.

The term 'insulin resistance in endocrine disease' refers to endocrine disorders associated with changes in sensitivity to insulin, often leading to high blood sugar. Examples are acromegaly, but also growth hormone deficiency, hypercortisolism in the course of Cushing's syndrome, hyper- or hypothyroidism, primary hyperparathyroidism, aldosteronism, pheochromocytoma, congenital hypertrophy of the adrenal glands, polycystic ovaries syndrome, hypogonadism, or other hormonally active neuroendocrine tumours. High blood sugar in insulin resistance in endocrine disease is often reversible, and may be reversed by insulin-sensitizing agents.

The term 'Polycystic Ovary Syndrome' (PCOS) refers to a full-body endocrine and metabolic condition that affects the ovaries and ovulation. PCOS is associated with profound insulin resistance as well as with defects in insulin secretion. Therefore, strategies to lower insulin resistance may lead to prevention and/or treatment of PCOS.

The term 'Nonalcoholic fatty liver disease' (NAFLD) refers to a group of conditions where there is accumulation of excess fat in the liver of people who drink little or no alcohol. The most common form of NAFLD is called fatty liver. NAFLD is strongly associated with insulin resistance and type 2 diabetes mellitus, therefore treatments of NAFLD may aim at lowering insulin resistance.

The term 'Nonalcoholic steatohepatitis' (NASH) refers to liver inflammation and damage caused by a buildup of fat in the liver. NASH is associated with a markedly increased risk of developing cirrhosis and hepatocellular carcinoma as well as other diseases not directly associated with liver damage, including increased risk of cardiovascular disease. An association between NASH and insulin resistance is well-known, and strategies to lower insulin resistance may decrease disease progression or symptoms in NASH.

### COMBINATION COMPRISING AT LEAST ONE INSULIN-SENSITIZING AGENT AND AT LEAST ONE BUTYRATE-PRODUCING BACTERIUM

The present disclosure provides a use that may improve the beneficial therapeutic effects of insulin-sensitizing agents in combination with one or more butyrate-producing bacteria, for use in the prevention or treatment of metabolic disease, insulin resistance and/or insulin resistance-related conditions preferably chosen from type 1 diabetes mellitus , type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

Moreover, the present disclosure relates to a combination for use comprising at least one insulin-sensitizing agents and at least one butyrate-producing bacteria, for use in the prevention or treatment of metabolic disease, insulin resistance and/or insulin resistance-related conditions, preferably chosen from type 1 diabetes mellitus , type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

In addition or alternative to preventing and/or treating metabolic disease, insulin resistance and/or insulin resistance-related conditions, the combination according to the present disclosure may be used for (enhancing) butyric acid and/or butyrate production, preferably *in situ*, i.e. in the small intestine. Similarly, the combination according to the present disclosure or butyrate-producing bacteria according to the present disclosure are also capable of decreasing the level of lactate, e.g. *in situ,* in the small intestine (lactate is known to be an undesired compound in the intestinal tract).

In particular, it was found that a combination wherein the insulin-sensitizing agent is sorbitol, and the butyrate-producing bacterium is *Anaerobutyricum soehngenii, (or Anaerobutyricum hallii*) or relative thereof having a 16S rRNA gene sequence with at least 97% sequence identity with SEQ ID NO:1 or SEQ ID NO:2, is particularly useful for (enhancing) butyric acid and/or butyrate production, preferably *in situ,* i.e. in the small intestine.

### Insulin-sensitizing agent

The term 'insulin-sensitizing agent' is used in current disclosure to describe an agent that reduces insulin resistance (e.g. together with the butyrate-producing bacterium according to the present disclosure), typically as measured by one of the herein preferred methods, such as the hyperinsulinemic euglycemic clamp, HOMA(-IR), and/or fasting serum insulin levels, e.g. in experimental research and/or clinical setting. The skilled person however also knows other methods of measuring insulin-sensitizing effects.

The insulin-sensitizing agent as taught in present disclosure may be selected from ingredients such as inositol, thiamine HCl, riboflavin, pyridoxine HCl, niacin, choline chloride, calcium pantothenate, biotin, folic acid, vitamin B12, p-aminobenzoic acid, vitamin A acetate, vitamin K, vitamin D, vitamin E, and the like), sugars and complex carbohydrates (e.g. water-soluble and water-insoluble monosaccharides, disaccharides, and polysaccharides), medicinal compounds (e.g. antibiotics, glibenclamide, pioglitazone, rosiglitazone, and biguanide), antioxidants, trace element ingredients (e.g. compounds of cobalt, copper, manganese, iron, zinc, tin, nickel, chromium, molybdenum, iodine, chlorine, silicon, vanadium, selenium, magnesium, sodium and potassium and the like).

The insulin-sensitizing agent according to the present disclosure is preferably chosen from one or more of chromium, inositol, metformin and/or sorbitol as disclosed herein.

### Chromium (picolinate)

Chromium has been identified as regulating insulin by increasing the sensitivity of the insulin receptor and/or by increasing the number of insulin receptors. Chromium may potentiate the actions of insulin, augment the insulin signaling pathway, blunt the negative-regulators of insulin signaling, enhance AMP-activated protein kinase (AMPK) activity, upregulate cellular glucose uptake, and/or attenuate oxidative stress.

Several alternate forms of chromium may lead to improved absorption of chromium by the body, which include but are not limited to chromium picolinate, chromium(III) picolinate, chromium histidinate, chromium phenylalanine, chromium yeast, chromium chloride, chromium nicotinate, chromium nicotinate-glycinate, chromium polynicotinate, and chromium citrate.

In particular chromium(III)picolinate, also called chromium tripicolinate, has been proposed as a treatment for metabolic disease, such as insulin resistance and/or insulin resistance-related conditions, including type 2 diabetes mellitus. Chromium(III) picolinate is a compound derived from chromium(III) and picolinic acid (also called coordination complex of chromium(III) and picolinic acid). As one of the several proposed mechanisms by which chromium(III) picolinate may be beneficial in the prevention or treatment of insulin resistance and/or insulin resistance-related conditions, absorbed chromium(III) picolinate may give up Cr3+ to transferrin. In turn, transferrin transports Cr3+ to insulin sensitive cells (i.e. adipocytes) where it binds to apochromodulin to form holochromodulin. Holochromodulin binds to the insulin receptor, which assists in maintaining the active conformation of the insulin receptor by prolonging the kinase activity of kinases or up-regulating the amount of insulin receptor mRNA levels.

The insulin-sensitizing agent which can be used in combination with a butyrate-producing bacterium as taught in the current disclosure may be chromium, chromium picolinate, chromium(III) picolinate, chromium histidinate, chromium phenylalanine, chromium yeast, chromium chloride, chromium nicotinate, chromium nicotinate-glycinate, chromium polynicotinate, and chromium citrate, most preferably chromium(III) picolinate. However, an alternative chromium form may be more beneficial depending on the clinical need, route of administration, and/or administration form.

### (myo-)inositol

Inositol (or cyclohexane-1,2,3,4,5,6-hexol) is a compound with formula C₆H₁₂O₆ or (-CHOH-)₆, a derivative of cyclohexane with six hydroxyl groups. An exemplary chemical formula is:

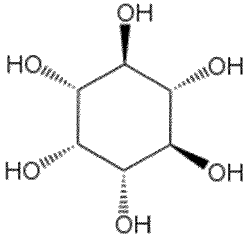

Inositol can be seen as a sugar alcohol with typically half the sweetness of sucrose, and exists in nine possible stereoisomers. The stereoisomers scyllo-inositol, muco-inositol, (D-)chiro-inositol, and neo-inositol, occur in relatively low quantities in nature.

Cis-1,2,3,5-trans-4,6-cyclohexanehexol, or myo-inositol (also referred to as meso-inositol or i-inositol), is the most widely occurring inositol stereoisomer in nature and is the most bioavailable. Dietary inositol supplements generally comprise the myo-inositol form. It is known that dietary myo-inositol supplementation can reduce insulin resistance, although the exact mechanism through which this occurs is, for at least a part, unknown. Myo-inositol's insulin-sensitizer effects could involve the generation of inositol triphosphate (IP3), phosphatidylinositol phosphate (PIP), phosphatidylinositol biphosphate (PIP2), and phosphatidylinositol triphosphate (PIP3). PIP3 acts as a second messenger in the insulin pathway through the activation of PI3K/AKT. On the other hand, the increase of phosphoinositides on the intracellular environment can exert important changes in energetic state of the cell that can be detected by energy sensor kinases as Liver kinase B1 (LKB1) and the AMP-activated protein kinase (AMPK).

The insulin-sensitizing agent which can be used in combination with a butyrate-producing bacterium as taught in the current disclosure may be inositol, myo-inositol, scyllo-inositol, muco-inositol, (D-)chiro-inositol, and neo-inositol, most preferably myo-inositol.

The current disclosure preferably uses the myo-inositol form, however other inositol isomers may be more beneficial depending on the clinical need and/or route of administration.

### Metformin

Metformin is a member of the class of guanidines that is biguanide, carrying two methyl substituents at position 1. It has a role as a hypoglycemic agent, a xenobiotic and an environmental contaminant. Metformin is known for use to lower the blood sugar in metabolic disease, such as insulin resistance and insulin resistance-related conditions, including type 2 diabetes mellitus. The exemplary formula of metformin is:

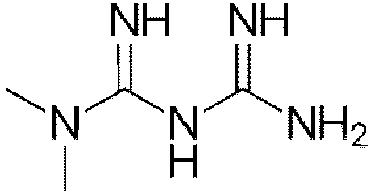

The molecular mechanism by which metformin prevents/treats metabolic disease is, at least for a part, not completely understood. Multiple potential mechanisms of action have been proposed: inhibition of the mitochondrial respiratory chain (complex I), activation of AMP-activated protein kinase (AMPK), inhibition of glucagon-induced elevation of cyclic adenosine monophosphate (cAMP) with reduced activation of protein kinase A (PKA), inhibition of mitochondrial glycerophosphate dehydrogenase, and regulation of gut microbiota. Metformin also exerts an anorexiant effect in most people, decreasing caloric intake. Ultimately, it decreases gluconeogenesis (glucose production) in the liver. It also has an insulin-sensitizing effect with multiple actions on tissues including the liver, skeletal muscle, endothelium, adipose tissue, and the ovaries. In the clinical setting, metformin is generally provided as metformin hydrochloride.

In the various embodiments of the present disclosure, the metformin may be any type or form of metformin. In the various embodiments of the present disclosure, the metformin is preferably metformin hydrochloride (1,1-dimethylbiguanide hydrochloride).

### Sorbitol

Sorbitol, also called D-sorbitol, 50-70-4, E420, or D-glucitol, is a type of carbohydrate. It falls into a category of sugar alcohols called polyols. Sorbitol has about one-third fewer calories and about 60% of the sweetening activity of sucrose and is used as a sugar replacement in diabetes. An exemplary formula of sorbitol is:

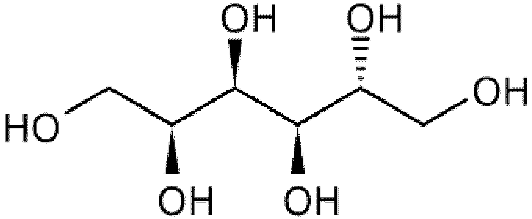

Sorbitol is generally obtained from potato starch, but it is also found in e.g. apples, pears, peaches, and prunes. Sorbitol is an isomer of mannitol, another sugar alcohol; the two differ only in the orientation of the hydroxyl group on carbon 2. While similar, the two sugar alcohols have very different sources in nature, melting points, and uses. Sorbitol can be converted to fructose by sorbitol-6-phosphate 2-dehydrogenase.

Unabsorbed sorbitol retains water in the large intestine through osmotic pressure and may thereby stimulate peristalsis of the intestine and exert a diuretic, laxative and/or cathartic effect.

In addition to or as an alternative to sorbitol as according to the present disclosure, also melibiose, pyruvate, lactose or lactulose may be used.

### Butyrate-producing bacterium

The present inventors surprisingly found that the co-administration of a butyrate-bacterium together with an insulin-sensitizing agent according to the present disclosure may provide a synergistic therapeutic effect in the prevention or treatment of metabolic disease, insulin resistance and/or insulin resistance-related conditions, preferably chosen from type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

A possible mechanisms resulting in the beneficial effect disclosed by current inventors, may be that the one or more butyrate-producing bacterium improves the insulin-sensitizing effect of the insulin-sensitizing agent.

Subjects suffering a metabolic disease, insulin resistance, and/or insulin resistance-related condition, typically have reduced levels of bacteria that produce short-chain fatty acids (SCFA), in particular butyrate (Karlsson et al. Nature. 2013 Jun 6;498(7452):99-103).

It is known that the transplantation of fecal microbiota comprising butyrate-producing bacteria may be used for treating intestinal dysbiosis and pathologies associated with such dysbiosis, including diabetes type 2 mellitus (WO 2014/121298 A2). The administration of selective butyrate-producing strains may therefore have therapeutic value.

To illustrate, WO 2016/110585 A1 discloses isolated intestinal bacterial strains such as *Intestinimonas butyriciproducens* (*I. butyriciproducens),* comprising a lysine pathway gene set, being capable of converting L-lysine into butyric acid and/or butyrate or a derivative thereof. WO 2021/028585 A1 discloses butyrate-bacteria from from the *Anaerostipes* genus, most preferably *Anaerostipes rhamnosivorans (A. rhamnosivorans),* comprising a gene set encoding an inositol to propionic acid pathway. WO 2019/046372 A1 discloses the use of *Eubacterium hallii* (later renamed to *Anaerobuturicum soehngenii* and *Anaerobutyricum hallii -* see below) for treating insulin resistance and insulin resistance-related diseases.

The terms 'butyrate-producing bacterium' or 'butyric acid-producing bacterium' refer to a bacterium which is capable of producing butyric acid and/or butyrate and/or one or more derivatives thereof. A prominent pathway by which butyric acid and/or butyrate and derivative thereof may be produced *in situ* in the mammalian gut (or *in vitro* in culture), i.e. a butyric acid/butyrate synthesis pathway gene set, is the so-called 'acetyl-CoA pathway'. The acetyl-CoA pathway has been well-documented and is known to be particularly prevalent in intestinal bacteria belonging, for instance, to the genus *Lachnospiraceae* and *Ruminococcaceae* (which together may form up to 20% of total gut microbiota). According to the acetyl-CoA pathway, butyric acid and/or butyrate and/or derivatives thereof may be formed by a single bacterial species via carbohydrate fermentation and/or by a group of microorganisms where metabolites from other organisms act as a substrate for butyrate-producing bacteria. The conventional acetyl-CoA pathway involves a cascade of enzymes, including (among many others) two key enzymes referred to as butyryl-CoA transferase (But) and butyrate kinase (Buk).

The term 'butyrate' or 'butyric acid' (also known under the systematic name butanoic acid) as used herein refers to a carboxylic acid with the structural formula CH₃CH₂CH₂COOH. The term may include derivatives thereof, i.e. compounds derived from butyric acid and includes salts and esters of butyric acid, which are known as butyrate or butanoate. Non-limiting examples of butyrate salts include sodium butyrate, calcium butyrate, magnesium butyrate, manganese butyrate, cobalt butyrate, barium butyrate, lithium butyrate, zinc butyrate, potassium butyrate, ferrous butyrate and the like. Non-limiting examples of butyrate esters (i.e. esters of butyric acid) include cellulose acetate butyrate, methyl butyrate, ethyl butyrate, butyl butyrate, pentyl butyrate, and the like.

The term 'propionic acid' as used herein refers is a carboxylic acid with chemical formula CH₃CH₂CO₂H. The term may include derivatives thereof, i.e. compounds derived from propionic acid and in particular salts and esters of propionic acid which are known as propionates or propanoates. Non-limiting examples of propionate salts are ammonium propionate, calcium propionate, magnesium propionate, potassium propionate and sodium propionate. Non-limiting example of propionate ester is ethyl propionate.

Without wishing to be bound by any theories, it is believed that the butyrate-producing bacterium according to the present disclosure, when administered to a human being or when ingested by a human being in an adequate amount, is able to survive and at least transiently colonize the gastrointestinal tract of said human being. This colonization may typically enable greater *in situ* production of butyric acid/butyrate, although other mechanisms cannot be excluded. Increased *in situ* production may underlie, at least in part, the beneficial effects in the combination as taught herein, e.g. preventing and/or treatment of metabolic disease, such as insulin resistance and insulin resistance-related conditions, chosen from type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

Preferably, the butyrate-producing bacterium according to the present disclosure belongs to the phylum *Firmicutes*, preferably to the taxon Clostridium (cluster XIV). In an embodiment, the butyrate-producing bacterium taught herein may be isolated from a human intestine, i.e., is a human intestinal isolate.

The butyrate-producing bacterium according to the present disclosure is preferably chosen from:
- *Anaerobutyricum* species or *Eubacterium* species, preferably *Anaerobutyricum soehngenii* (e.g. DSM17630/KCTC15707) and/or *Anaerobutyricum hallii* (DSM3353/ATCC27751);
- *Intestinimonas* species, preferably *Intestinimonas butyriciproducens* (e.g. DSM26588); and/or
- *Anaerostipes* species, preferably *Anaerostipes rhamnosivorans* (e.g. DSM26241).

### Anaerobutyricum soehngenii and/or Anaerobutyricum hallii

In a study by Shetty et al (Int J Syst Evol Microbiol. 2018 Dec;68(12):3741-3746), the species formerly known as *Eubacterium hallii* has been reclassified into two groups: *Anaerobutyricum hallii* and *Anaerobutyricum soehngenii.* Both *Anaerobutyricum soehngenii* and/or *Anaerobutyricum hallii* are considered as an anaerobic Gram-positive, catalase-negative bacterium belonging to the clostridial cluster XIVa of the phylum Firmicutes.

Most preferably the *Anaerobutyricum* species according to the present disclosure is *Anaerobutyricum soehngenii* (e.g. DSM17630/KCTC15707), or a relative thereof having a 16S rRNA gene sequence with at least 70, 80, 85, 90, 95, 96, 97, 98, 99, 99.5, 99.9% sequence identity with the 16S rDNA sequence of *Anaerobutyricum soehngenii* (SEQ ID NO:1). Such cut-off value based on 16S rDNA similarity can define species with similar characteristics and/or functionality.

In addition or alternatively, the *Anaerobutyricum* species according to the present disclosure is *Anaerobutyricum hallii* (e.g. DSM3353/ATCC27751), or a relative thereof having a 16S rRNA gene sequence with at least 70, 80, 85, 90, 95, 96, 97, 98, 99, 99.5, 99.9% sequence identity with the 16S rDNA sequence of *Anaerobutyricum hallii* (SEQ ID NO:2). Such cut-off value based on 16S rDNA similarity can define species with similar characteristics and/or functionality.

| *Anaerobutyricum soehngenii L2-7* 16S rRNA sequence |
|---|
| Nucleotide sequence (SEQ ID NO:1)* |
| tgatcctggc tcaggatgaa cgctggcggc gtgcctaaca catgcaagtc gaacgaagca ccttttaaga ttcttcggat gattgatcgg tgactgagtg gcggacgggt gagtaacgcg tgggtaacct gccctgtaca gggggataac agttggaaac ggctgctaat accgcataag cgcacgagag gacatcctct tgtgtgaaaa actccggtgg tacaggatgg gcccgcgtct gattagctgg ttggcagggt aacggcctac caaggcgacg atcagtagcc ggtctgagag gatgaacggc cacattggaa ctgagacacg gtccaaactc ctacgggagg cagcagtggg gaatattgca caatggggga aaccctgatg cagcaacgcc gcgtgagtga agaagtattt cggtatgtaa agctctatca gcagggaaga taatgacggt acctgactaa gaagctccgg ctaaatacgt gccagcagcc gcggtaatac gtatggagca agcgttatcc ggatttactg ggtgtaaagg gtgcgtaggt ggcagtgcaa gtcagatgtg aaaggccggg gctcaacccc ggagctgcat ttgaaactgc atagctagag tacaggagag gcaggcggaa ttcctagtgt agcggtgaaa tgcgtagata ttaggaggaa caccagtggc gaaggcggcc tgctggactg ttactgacac tgaggcacga aagcgtgggg agcaaacagg attagatacc ctggtagtcc acgccgtaaa cgatgaatcc taggtgtcgg ggccgtatag gcttcggtgc cgtcgcaaac gcagtaagta ttccacctgg ggagtacgtt cgcaagaatg aaactcaaag gaattgacgg ggacccgcac aagcggtgga gcatgtggtt taattcgaag caacgcgaag aaccttacca ggtcttgaca tccttctgac cactccgtaa tgggagtctt ccttcgggac agaagagaca ggtggtgcat ggttgtccgt cagctcgtgt cgtgagatgt tgggttaagt cccgcaacga gcgcaacccc tatcttcagt agccagcagg taaggctggg cactctggag agactgccag ggataacctg gaggaaggtg gggacgacgt caaatcatca tgccccttat gatctgggcg acacacgtgc tacaatggcg gtcacaaagt gaggcaaacc tgcgaggggg agcaaaccac aaaaaggccg tcccagttcg gactgtagtc tgcaacccga ctacacgaag ctggaatcgc tagtaatcgc gaatcagaat gtcgcggtga atacgttccc gggtcttgta cacaccgccc gtcacaccat gggagtcgga aatgcccgaa gccagtgacc caaccttttg gagggarctg tcgaaggtgg agccggtaac tggggtgaag tcgtaacaag gg |

| *Anaerobutyricum hallii* 16S rRNA sequence |
|---|
| Nucleotide sequence (SEQ ID NO:2)* |
| tttatttgag agtttgatcc tggctcagga tgaacgctgg cggcgtgcct aacacatgca agtcgaacga agcaccttac cwgattcttc ggatgaaagw ytggtgactg agtggcggac gggtgagtaa cgcgtgggta acctgccctg tacaggggga taacagctgg aaacggctgc taataccgca taagcgcacg aggagacatc tccttgtgtg aaaaactccg gtggtacagg atgggcccgc gtctgattag ctggttggca gggtaacggc ctaccaaggc aacgatcagt agccggtctg agaggatgaa cggccacatt ggaactgaga cacggtccaa actcctacgg gaggcagcag tggggaatat tgcacaatgg gggaaaccct gatgcagcaa cgccgcgtga gtgaagaagt atttcggtat gtaaagctct atcagcaggg aagataatga cggtacctga ctaagaagct ccggctaaat acgtgccagc agccgcggta atacgtatgg agcaagcgtt atccggattt actgggtgta aagggtgcgt aggtggcagt gcaagtcaga tgtgaaaggc cggggctcaa ccccggngct gcatttgaaa ctgcwyrgct agagtacagg agaggcaggc ggaattccta gtgtagcggt gaaatgcgta gatattagga ggaacaccag tggcgaaggc ggcctgctgg actgttactg acactgaggc acgaaagcgt ggggagcaaa caggattaga taccctggta gtccacgccg taaacgatga atactaggtg tcggggccgt ataggctycg gtgccgccgc taacgcagta agtattccac ctggggagta cgttcgcaag aatgaaactc aaaggaattg acggggaccc gcacaagcgg tggagcatgt ggtttaattc gaagnaacgc gaagaacctt accaggtctt gacatccttc tgaccgcacc ttaatcggtg ctttccttcg ggacagaaga gacaggtggt gcatggttgt cgtcagctcg tgtcgtgaga tgttgggtta agtcccncaa cgagcgcnac ccctatcttc agtagccagc aggtaaggct gggcactctg gagagactgc cagggataac ctggaggaag gtggggacga cgtnnaatca tcatgcccct tatgatctgg gcgacacacg tgctacnatg gcggtcacag agtgaggcga accygcgang gggagcaanc cacaaaaagg ccgtcccagt tcggactgta gtctgcaacc cgactacacg |
| aagctggaat cgctagtaat cgcgaatcag aatgtcgcgg tgaatacgtt cccnngtctt gtacacaccg nccgtcacac catgggagtc ggaaatgccc gaagccagtg acccaacctt tatggaggga gctgtcgaag gtggagccgg taactgggg |
| |
| *"n" refers to a, t, c, or g. |

### Intestinimonas butyriciproducens

The butyrate-producing bacterium according to the present disclosure may be an *Intestinimonas* species, preferably *Intestinimonas butyriciproducens* (e.g. DSM26588).

Said *Intestinimonas* species may be capable of converting L-lysine into butyric acid and/or butyrate or a derivative thereof. Said *Intestinimonas* species may be capable of converting L-lysine into butyric acid and/or butyrate or a derivative thereof and acetate or a derivative thereof.

The *Intestinimonas* species taught herein may belong to the phylum *Firmicutes*, the taxon Clostridium cluster IV, the genus *Intestinimonas,* and preferably belongs to the species *Intestinimonas butyriciproducens.*

Most preferably the *Intestinimonas* species according to the present disclosure is *Intestinimonas butyriciproducens* (e.g. DSM26588), or a relative thereof having a 16S rRNA gene sequence with at least 70, 80, 85, 90, 95, 96, 97, 98, 99, 99.5, 99.9% sequence identity with the 16S rDNA sequence of *Intestinimonas butyriciproducens* (SEQ ID NO:3). Such cut-off value based on 16S rDNA similarity can define species with similar characteristics and/or functionality.

| *Intestinimonas butyriciproducens* AF211 16S rRNA sequence |
|---|
| Nucleotide sequence (SEQ ID NO:3)* |
| gtgcagtcga cggagcaccc ctgacggagt tttcggacaa cgaaagggaa tgcttagtgg cggacgggtg agtaacgcgt gagtaacctg ccttggagtg gggaatagca gccggaaacg gctgctaata ccgcatgatg tatctggatc gcatggttct ggataccaaa gatttattgc tctgagatgg actcgcgtct gattagctag ttggtgaggt aacggctcac caaggcgacg atcagtagcc ggactgagag gttggccggc cacattggga ctgagacacg gcccagactc ctacgggagg cagcagtggg gaatattggg caatgggcga aagcctgacc cagcaacgcc gcgtgaagga agaaggccct cgggttgtaa acttcttttg tcagggacga agcaagtgac ggtacctgac gaataagcca cggctaacta cgtgccagca gccgcggtaa tacgtaggtg gcaagcgtta tccggattta ctgggtgtaa agggcgtgta ggcgggagtg caagtcagat gtgaaaacta tgggctcaac ccatagcctg catttgaaac tgtacttctt gagtgatgga gaggcaggcg gaattccctg tgtagcggtg aaatgcgtag atatagggag gaacaccagt ggcgannncg gcctgctgga cattaactga cgctgnnncg cgaaagcgtg gggagcaaac aggattagat accctggtag tccacgccgt aaacgatgga tactaggtgt ggggggtctg accccctccg tgccgcagtt aacacaataa gtatcccacc tggggagtac gatcgcaagg ttgaaactca aaggaattga cgggggcccg cacaagcggt ggagtatgtg gtttaattcg aagcaacgcg aagaacctta ccaggacttg acatcctact aacgaagcag agatgcataa ggtgcccttc ggggaaagta gagacaggtg gtgcatggtt gtcgtcagct cgtgtcgtga |
| gatgttgggt taagtcccgc aacgagcgca acccttattg ttagttgcta cgcaagagca ctctagcgag actgccgttg acaaaacgga ggaaggtggg gacgacgtca aatcatcatg ccccttatgt cctgggccac acacgtacta caatggcggt caacagaggg aagcaaagcc gcgaggtgga gcaaatccct aaaagccgtc ccagttcgga ttgcaggctg aaactcgcct gtatgaagtc ggaatcgcta gtaatcgcgg atcagcatgc cgcggtgaat acgttcccgg gccttgtaca caccgcccgt cacaccatga gagtcgggaa cacccgaagt ccgtagccta acagcaatgg ggcgcggccg aagga |
| |
| *"n" refers to a, t, c, or g. |

In a preferred embodiment, the *Intestinimonas* species according to the present disclosure, preferably *Intestinimonas butyriciproducens* (e.g. DSM26588), comprises a lysine pathway gene set that allows said *Intestinimonas* species to convert L-lysine into butyric acid and/or butyrate or a salt or ester thereof, and further may comprise a fructose-lysine uptake and degradation operon that allows said *Intestinimonas* species to convert fructose-lysine into butyric acid and/or butyrate or a salt or ester thereof, wherein preferably:
- the lysine pathway gene set comprises genes encoding the proteins: Lysine 2,3-aminomutase; L-beta-lysine 5,6-aminomutase alpha subunit; L-beta-lysine 5,6-aminomutase beta subunit; 3,5-diaminobexanoate dehydrogenase; 3-keto-5-aminohexanoate cleavage enzyme; 3-aminobutyryl-CoA ammonia-lyase; butyrate-acetoacetate CoA-transferase subunit A; butyrate-acetoacetate CoA-transferase subunit B; acetyl-CoA:acetoacetyl-CoA transferase, and/or wherein
- the fructose-lysine uptake and degradation operon comprises the genes encoding the proteins: fructose-lysine kinase; fructosamine deglycase; ABC transporter periplasmic spermidine putrescine-binding protein PotD; spermidine putrescine ABC transporter permease component PotC; spermidine putrescine ABC transporter permease component PotB; putrescine transport ATP-binding protein PotA, and optionally fructose-lysine 3-epimerase.

Accordingly, in addition or instead of the *Intestinimonas* species as taught herein, any bacterium that comprises said lysine pathway gene set and/or (fructose-)lysine uptake and degradation operon can be used in the present disclosure. For example, a bacterium of choice may be transfected with the lysine pathway gene set and/or the fructose-lysine uptake and degradation operon as taught herein, and which accordingly will be capable of converting L-lysine into butyric acid and/or butyrate or a derivative thereof and/or is capable of converting fructose-lysine into butyric acid and/or butyrate or a derivative thereof. The skilled person is well-acquainted with methods for transfecting bacteria with a desired genetic construct (e.g. operon and/or pathway gene set).

Without wishing to be bound to any theories, it is believed that the *Intestinimonas* species as taught herein, or any bacterium that comprises said lysine pathway gene set and/or (fructose-)lysine uptake and degradation operon, when administered to a human being or when ingested by a human being in an adequate amount, is able to colonize the GI tract of said human being. This colonization may, among others, enable greater *in situ* production of butyric acid and/or butyrate or a derivative thereof as, and/or greater metabolism of fructose-lysine or other glycated lysine in the GI tract of said human being.

### Anaerostipes rhamnosivorans

The butyrate-producing bacterium according to the present disclosure may be an *Anaerostipes* species, preferably *Anaerostipes rhamnosivorans* (e.g. DSM26241) or *Anaerostipes rhamnosivorans 1y2.*

Most preferably the *Anaerostipes* species according to the present disclosure is *Anaerostipes rhamnosivorans* (e.g. DSM26241), or a relative thereof having a 16S rRNA gene sequence with at least 70, 80, 85, 90, 95, 96, 97, 98, 99, 99.5, 99.9% sequence identity with the 16S rDNA sequence of *Anaerostipes rhamnosivorans* (SEQ ID NO:4). Such cut-off value based on 16S rDNA similarity can define species with similar characteristics and/or functionality.

| *Anaerostipes rhamnosivorans 1y-2* 16S rRNA sequence |
|---|
| Nucleotide sequence (SEQ ID NO:4)* |
| tattttggat tgaagttttc ggatggatct ccttaatgac tgagtggcgg acgggtgagt aacgcgtggg gaacctgccc tatacagggg gataacagct ggaaacggct gctaataccg cataagcgca cagaatcgca tgattcagtg tgaaaagccc tggcagtata ggatggtccc gcgtctgatt agctggttgg cggggtaacg gcccaccaag gcgacgatca gtagccggct tgagagagtg gacggccaca ttgggactga gacacggccc aaactcctac gggaggcagc agtggggaat attgcacaat gggggaaacc ctgatgcagc gacgccgcgt gagtgaagaa gtatttcggt atgtaaagct ctatcagcag ggaagaaata agacggtacc tgactaagaa gccccggcta actacgtgcc agcagccgcg gtaatacgta gggggcaagc gttatccgga attactgggt gtaaagggtg cgtaggtggc atgataagtc agaagtgaaa gcccggggct taaccccggg actgcttttg aaactgtaat gctagagtgc aggagaggta agcggaattc ctagtgtagc ggtgaaatgc gtagatatta ggaggaacac cagtggcgaa ggcggcttac tggactgtca ctgacactga ggcacgaaag cgtggggagc aaacaggatt agataccctg gtagtcnacg ccgtaaacga tgaatactag gtgtcggggc cgtagaggct tcggtgccgc agcaaacgca gtaagtattc cacctgggga gtacgttcgc aagaatgaaa ctcaaaggaa ttgacgggga cccgcacaag cggtggagca tgtggtttaa ttcgaagcaa cgcgaagaac cttacctggt cttgacatcc ttctgaccgg ttnnnaaccg aacctttcct tcgggacaga agtgacaggt ggtgcatggt tgtcgtcagc tcgtgtcgtg agatgttggg ttaagtcccg caacgagcgc aacccctatc tttagtagcc agcatataag gtgggcactc tagagagact gccagggata acctggagga aggtggggac gacgtcaaat catcatgccc cttatggcca gggctacaca cgtgctacaa tggcgtaaac aaagggaagc gaccccgcga gggcaagcaa atcccagaaa taacgtctca gttcggattg tagtctgcaa ctcgactaca tgaagctgga atcgctagta atcgtgaatc agaatgtcac ggtgaatacg ttcccgggtc ttgtacacac cgcccgtcac accatgggag tcagtaacgc ccgaagtcag tgacccaacc gcaaggagga ga |
| |
| *"n" refers to a, t, c, or g. |

In a preferred embodiment, the *Anaerostipes* species comprises a gene set encoding an inositol to propionic acid pathway that allows said *Anaerostipes* species to convert (myo- and/or chiro-) inositol to propionic acid or a salt or ester thereof, in particular but not necessarily under anaerobic conditions, such as wherein the bacterium and/or its medium is not in contact with gas comprising more than 1, 2, 3, 4, 5, 10 vol% oxygen. The *Anaerostipes* species is preferably, but not necessarily, an isolate, e.g. a human intestinal isolate.

In a preferred embodiment, the level of the *Anaerostipes* species in the GI tract of a subject may be increased by administering the combination as disclosed herein, comprising at least one insulin-sensitizing agent and an *Anaerostipes* species bacterium as the at least one butyrate-producing bacterium, preferably *Anaerostipes rhamnosivorans* 1y2. Alternatively, in addition or instead to the *Anaerostipes* species, a bacterium may be used which has been transfected with the inositol to propionic acid pathway gene set as taught herein, and which is capable of converting inositol, for example myo-inositol and/or chiro-inositol, to propionic acid or a derivative thereof. The skilled person is well-acquainted with methods for transforming bacteria with a desired genetic construct (i.e. pathway gene set).

In an embodiment, the *Anaerostipes* species according to the present disclosure belongs to the phylum *Firmicutes,* preferably to the class Clostridia (and/or order Clostridiales), more preferably to the family Lachnospiraceae, more preferably to the genus *Anaerostipes,* even more preferably to the species *Anaerostipes rhamnosivorans.* Alternatively, the *Anaerostipes* species according to the present disclosure is *Anaerostipes butyraticus,* preferably type strain DSM22094.

In an embodiment, the present disclosure is concerned with a combination comprising a butyrate-producing bacterium as taught herein that may be for use as a probiotic. Accordingly, 'probiotics' as used herein refers to microorganisms such as intestinal bacteria, which - when administered or ingested in effective amounts - confer health benefits to the host (e.g. humans or mammals). Preferably, probiotics should be alive or viable when administered to a subject so as to allow the probiotics to colonize the large intestine of the host. However, under certain conditions, probiotics may also be dead when administered provided that substances produced by the probiotics still exert probiotic, beneficial effects on the host. Most probiotics or probiotic products are composed of lactic acid bacteria such as *Lactobacilli* or *Bifidobacteria.* The skilled person is well-acquainted with the field of probiotics and knows how to select lactic acid bacteria endowed with probiotic activity.

In an embodiment, the present combination as taught herein may be for use as a symbiotic. The term 'symbiotic' or 'symbiotic products' as used herein generally refers to compositions and/or nutritional supplements combining probiotics and one or more compounds that promote the growth and/or activity of GI microorganisms, such as prebiotics, into one product. The symbiotic beneficially affects the host by improving the survival and colonization of the probiotic in the GI tract, by selectively stimulating the growth and/or by activating the metabolism of the probiotic, thus improving host welfare. The skilled person is well-acquainted with symbiotics and knows how to select ingredients that may be combined into a symbiotic.

The present inventors furthermore surprisingly found that micro-encapsulation of the butyrate-producing bacterium according to the present disclosure, i.e. co-administration of a micro-encapsulated butyrate-bacterium together with an insulin-sensitizing agent according to the present disclosure, may provide a further synergistic therapeutic effect in the prevention or treatment of metabolic disease, insulin resistance and/or insulin resistance-related conditions, preferably chosen from type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

A possible mechanism resulting in the further enhanced beneficial effect of micro-encapsulated butyrate-producing bacteria in combination with insulin-sensitizing agents, may be that the micro-encapsulated butyrate-producing bacterium further improves the insulin-sensitizing effect of the insulin-sensitizing agent.

The term 'micro-encapsulation' is used to describe the encapsulation of bacteria in a matrix, coating, or membrane, generally a protective matrix or protective membrane. The (average) diameter of the microcapsules may be between 50 nm and 2 mm, preferably between 100 nm and 1 mm. The matrix, coating or membrane is typically comprised of milk, milk protein, and/or a polymer. The purpose of micro-encapsulation, among other possible purposes, may be to protect bacteria and their components against destruction by the surrounding environment, such as the gastrointestinal environment. The micro-encapsulation of bacteria may also support improved incorporation of bacteria into dairy products, food products, pharmaceutical formulations, and/or pharmaceutical compositions. The micro-encapsulation of bacteria may also support the therapeutic effect. The micro-encapsulation of butyrate-producing bacteria may also improve the insulin-sensitizing effect of butyrate-producing bacteria in combination with insulin-sensitizing agents.

Various materials may be used for the micro-encapsulation of bacteria, such as pea protein, milk, milk protein, whey protein, casein, xanthan gum, alginate, gelatin, chitosan, carboxymethyl cellulose, starch, and/or carrageenan, and combinations thereof. In a preferred embodiment, the butyrate-producing bacteria as taught in the present combination is micro-encapsulated in one or more polymers. In a preferred embodiment, the butyrate-producing bacteria as taught in the present combination is micro-encapsulated in alginate. In another preferred embodiment, the butyrate-producing bacteria as taught in the present combination is micro-encapsulated in alginate and/or chitosan.

### EMBODIMENTS OF PRESENT DISCLOSURE

### Combination for use comprising an insulin-sensitizing agent and a butyrate-producing bacterium

The present disclosure teaches a combination for use comprising at least one insulin-sensitizing agent and at least one butyrate-producing bacterium in the treatment and/or prevention of metabolic disease, such as insulin resistance and insulin resistance-related conditions, preferably chosen from type 1 diabetes mellitus, type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

The subject receiving the combination as taught herein may be selected from the group consisting of human being, non-human primate, mouse, rat, dog, cow, and pig. In a preferred embodiment, the subject is a human.

The butyrate-producing bacterium as taught in the present combination may be comprised in the combination in an amount ranging from 10⁴ to 10¹⁵ colony forming units (CFU). For instance, the butyrate-producing bacterium may be comprised in the combination in an amount of 10⁶ CFU to 10¹³ CFU, preferably 10⁷ CFU to 10¹² CFU, preferably 10⁸ CFU to 10¹¹ CFU, more preferably 10⁹ CFU to 10¹¹ CFU, e.g. per dose or per ml or per g of formulation or composition comprising said.

In one of the embodiments, the bacterium in the combination taught herein may be incorporated in lyophilized form, micro-encapsulated form (reviewed by, for example, Solanki et al. BioMed Res. Int. 2013, Article ID 620719), or any other form preserving the activity and/or viability of the bacterial strain.

In an embodiment, the combination as taught herein may comprise one or more ingredients which are suitable for promoting survival and/or viability of the bacterium or strain derived therefrom as taught herein during storage and/or during exposure to bile and/or during passage through the GI tract of a mammal (e.g. a human being). Non-limiting examples of such ingredients include an enteric coating, and controlled release agents allowing passage through the stomach. The skilled person knows how to select suitable ingredients for maintaining a bacterium as taught herein viable and functional i.e. able to carry out intended function(s).

It may be advantageous to add one or more prebiotic ingredients to the combination as taught herein, for example, to supplement the effects (e.g. production of propionic acid/propionate and/or butyric acid/butyrate or a derivative thereof) of the bacterium as taught herein. The prebiotic ingredients may also enhance the activity and/or stimulate the growth of the bacterium, or a strain derived therefrom, as taught herein. A 'prebiotic' as used herein generally refers to a non-digestible food ingredient that promotes the growth of beneficial microorganisms in the intestines. Prebiotics or prebiotic products consist mainly of fermentable fibres or non-digestible carbohydrates. The fermentation of these fibres by probiotics promotes the production of beneficial end products, such as SCFAs, particularly butyrate. Non-limiting examples of suitable prebiotics include fibres such as inulin, pectin, and resistant starch, as well as cellobiose, maltose, mannose, salicine, trehalose, amygdalin, arabinose, melibiose, sorbitol, rhamnose and/or xylose. The skilled person is well-acquainted with the field of prebiotics and knows how to select ingredients endowed with prebiotic activity.

The (myo-)inositol or (myo-)inositol source (or a phospholipid or phytic acid source) as taught in the present disclosure may be comprised in the combination in the range of 10 mg to 100 g, preferably from 100 mg to 50 g, more preferably from 500 mg to 10 g, most preferably from 1 g to 5 g, e.g. per dose or per ml or per g of formulation or composition comprising said. In a preferred embodiment of present disclosure, the inositol is myo-inositol administered between 10 mg to 100 g, preferably from 100 mg to 50 g, more preferably from 500 mg to 10 g, most preferably from 1 g to 5 g, e.g. per dose or per ml or per g of formulation or composition comprising said.

The chromium as taught in the present disclosure may be comprised in the range of from 10 µg ([mu]g) to 5000 µg, more preferably from 50 µg to 2000 µg, and most preferably from 200 µg to 1000 µg. In a preferred embodiment of present disclosure, the chromium is chromium(III) picolinate administered between 10 µg and 5000 µg, or between 50 µg and 2000 µg, most preferably between 200 µg and 1000 µg, e.g. per dose or per ml or per g of formulation or composition comprising said.

The metformin as taught in the present disclosure may be any type or form of metformin. The metformin as taught in the present disclosure may be comprised in the range of 100 mg to 5000 mg, more preferably from 250 mg to 4000 mg, and most preferably from 1000 mg to 3000 mg, e.g. per dose or per ml or per g of formulation or composition comprising said. In a preferred embodiment of present disclosure, the metformin is metformin hydrochloride administered between 100 mg to 5000 mg, more preferably between 250 mg and 4000 mg, and most preferably between 1000 mg and 3000 mg, e.g. per dose or per ml or per g of formulation or composition comprising said.

The sorbitol as taught in the present disclosure may be comprised in the combination in the range of 10 mg to 100 g, preferably from 100 mg to 50 g, more preferably from 500 mg to 10 g, most preferably from 1 g to 5 g, e.g. per dose or per ml or per g of formulation or composition comprising said.

The insulin-sensitizing agent as taught herein may be administered separately, sequentially and/or simultaneously to the at least one insulin-sensitizing agent as taught herein.

In an embodiment of the current disclosure, the insulin-sensitizing agent and the butyrate-producing bacterium are administered in separate formulations, whereby there may be a separation in time between the administration of the insulin-sensitizing agent and the butyrate-producing bacterium.

In another embodiment of the current disclosure, the insulin-sensitizing agent and the butyrate-producing bacterium are administered at the same time in a separate formulation.

In yet another embodiment of the current disclosure, the insulin-sensitizing agent and the butyrate-producing bacterium are administered at the same time in the same formulation, or as a composition.

The term 'formulation' as used herein refers to the final product form of the insulin-sensitizing agent and the butyrate-producing bacterium when they are administered separately. The formulations possibly comprise additional ingredients, such as a carrier as disclosed herein. The formulations comprising the insulin-sensitizing agent and the formulations comprising the butyrate-producing bacterium may either or not have the same additional ingredients.

The term 'composition' as used herein refers to the final product form in which the insulin-sensitizing agent and butyrate-producing bacterium are both incorporated when the combination is administered simultaneously in a single composition. The composition possibly comprises additional ingredients, such as a carrier as disclosed herein.

The skilled person, and the (authorized) medical practitioner, is familiar with suitable administration formulations and appropriate administration timing of the insulin-sensitizing agent and the butyrate-producing bacterium as taught in the present disclosure. Moreover, the skilled person, and the (authorized) medical practitioner, is familiar with suitable methods to determine an appropriate administration formulation and appropriate administration timing of the insulin-sensitizing agent and the butyrate-producing bacterium.

In one of the embodiments, the butyrate-producing bacterium in the combination taught herein may be in a micro-encapsulated form.

The butyrate-producing bacterium as taught in the present combination may be micro-encapsulated by various means. The skilled person is aware of the various materials that may be used for the micro-encapsulation of bacteria, e.g. pea protein, milk, milk protein, whey protein, casein, xanthan gum, alginate, gelatin, chitosan, carboxymethyl cellulose, starch, and/or carrageenan, and combinations thereof. In a preferred embodiment, the butyrate-producing bacteria as taught in the present combination may be micro-encapsulated in a polymer. In a preferred embodiment, the butyrate-producing bacteria as taught in the present combination is micro-encapsulated in alginate. In another preferred embodiment, the butyrate-producing bacteria as taught in the present combination is micro-encapsulated in alginate and a second protein and/or polymer. In a more preferred embodiment, the butyrate-producing bacteria as taught in the present combination is micro-encapsulated in alginate and chitosan.

The butyrate-producing bacterium as taught in the present invention may be micro-encapsulated in two or more materials that are combined. Alternatively, the butyrate-producing bacterium as taught in the present invention may be micro-encapsulated consecutively in two or more materials. The butyrate-producing bacterium as taught in the present combination may or may not be lyophilized after micro-encapsulation. The butyrate-producing bacterium as taught in the present combination may or may not be lyophilized before micro-encapsulation.

### Combinations comprising formulations of an insulin-sensitizing agent and a butyrate-producing bacterium

In an embodiment of the combination for use, the insulin sensitizing agent and/or the butyrate-producing bacterium may be comprised in a food formulation, feed formulation, feed supplement formulation, food supplement formulation or pharmaceutical formulation. At the same time or alternatively, the insulin sensitizing agent and/or the butyrate-producing bacterium may be comprised in a liquid, liquid beverage (including dairy beverage and fermented beverage), yogurt, cheese, gel, gelatine, gelatine capsule, powder, paste, tablet, or a capsule.

The food or food supplement formulation is preferably a dairy product, more preferably a fermented dairy product, most preferably a yogurt or a yogurt drink.

The pharmaceutical formulation may be for example a liquid or solid form, more preferably a solid form solid dosage form, e.g., may be a capsule, a tablet, or a powder. Preferably, a pharmaceutical formulation does not relate to pure water or aqueous medium comprising more than 99 wt.% water.

The formulations as taught herein comprising the combination for use according to the present disclosure may further comprise any acceptable carrier that is suitable for keeping the butyrate-producing bacterium as taught herein viable until consumption by a subject (e.g. human or animal). For instance, non-limiting examples of acceptable carriers that are suitable for this purpose include any of well-known physiological or pharmaceutical carriers, buffers, and excipients. It will be appreciated that the choice for a suitable physiological or pharmaceutical carrier will depend upon the intended mode of administration of the formulations as taught herein (e.g. oral) and the intended form of the formulations (e.g. beverage, yogurt, powder, capsules, and the like). The skilled person knows how to select a physiological or pharmaceutical carrier, which is suitable for the formulations as taught herein.

The butyrate-producing bacterium as taught in the present disclosure may be comprise in the formulation in an amount ranging from 10⁴ to 10¹⁵ colony forming units (CFU). For instance, the butyrate-producing bacterium may be comprised in the combination in an amount of 10⁶ CFU to 10¹³ CFU, preferably 10⁷ CFU to 10¹² CFU, preferably 10⁸ CFU to 10¹¹ CFU, more preferably 10⁹ CFU to 10¹¹ CFU, e.g. per dose or per ml or per g of formulation or composition comprising said. Alternatively, the amount of butyrate-producing bacterium and/or administration frequency is chosen such that it is between, 10⁸ to 10¹³, preferably 10⁷ to 10¹², preferably 10⁸ to 10¹¹, more preferably 10⁹ to 10¹¹, all in CFU per day.

The (myo-)inositol or (myo-)inositol source (or a phospholipid or phytic acid source) as taught in the present disclosure may be comprised in a formulation in the range of from 55 µmol ([mu]mol) to 550 mmol, preferably from 550 µmol to 280 mmol, more preferably from 2.8 mmol to 55 mmol, most preferably 5.5 mmol to 28 mmol, or for example comprising from 10 mg to 100 g, preferably from 100 mg to 50 g, more preferably from 500 mg to 10 g, most preferably from 1 g to 5 g, e.g. per dose or per ml or per g of composition comprising said. Alternatively, the amount of inositol and/or administration frequency is chosen such that it is between 0.01 to 100, preferably from 0.1 to 50, more preferably from 0.5 to 10, most preferably from 1 to 5, all in grams per day.

In an embodiment for use according to the present disclosure, the (myo-)inositol can be formulated in the same composition as the at least one butyrate-producing bacterium, or the (myo-)inositol may be administered separately from the at least one butyrate-producing bacterium. In some cases, a subject can be on (myo-)inositol regimen already when the patient begins administration of the at least one butyrate-producing bacterium. In some cases, a patient can begin (myo-)inositol therapy at the same time as the patient begins administration of the at least one butyrate-producing bacterium. In some cases, a patient can begin (myo-)inositol therapy after the patient begins administration of the at least one butyrate-producing bacterium. The combination for use as taught herein may also be administered as a composition as disclosed herein.

The chromium as taught in the present disclosure may be comprised in a formulation in the range of from 24 nmol to 12 µmol ([mu]mol), more preferably from 120 nmol to 5 µmol, and most preferably from 500 nmol to 2.4 µmol, or for example comprising from 10 µg ([mu]g) to 5000 µg, more preferably from 50 µg to 2000 µg, and most preferably from 200 µg to 1000 µg, e.g. per dose or per ml or per g of composition comprising said. Alternatively, the amount of chromium and/or administration frequency is chosen such that it is between 10 and 5000, or between 50 and 2000, most preferably between 200 and 1000, all in µg (microgram) consumed per day.

In an embodiment for use according to the present disclosure, the chromium can be formulated in the same composition as the at least one butyrate-producing bacterium, or the chromium may be administered separately from the at least one butyrate-producing bacterium. In some cases, a subject can be on chromium regimen already when the patient begins administration of the at least one butyrate-producing bacterium. In some cases, a patient can begin chromium therapy at the same time as the patient begins administration of the at least one butyrate-producing bacterium. In some cases, a patient can begin chromium therapy after the patient begins administration of the at least one butyrate-producing bacterium. The combination for use as taught herein may also be administered as a composition as disclosed herein.

The metformin as taught in the present disclosure may be comprised in a formulation in the range of from 0.6 mmol to 30 mmol, more preferably from 1.5 mmol to 25 mmol, and most preferably from 6 mmol to 18 mmol, or for example comprising from 100 mg to 5000 mg, more preferably from 250 mg to 4000 mg, and most preferably from 1000 mg to 3000 mg, e.g. per dose or per ml or per g of composition comprising said. Alternatively, the amount of metformin and/or administration frequency is chosen such that it is between 100 to 5000, more preferably between 250 and 4000, and most preferably between 1000 and 3000, all in mg consumed per day.

In an embodiment for use according to the present disclosure, the metformin can be formulated in the same composition as the at least one butyrate-producing bacterium, or the metformin may be administered separately from the at least one butyrate-producing bacterium. In some cases, a subject can be on metformin regimen already when the patient begins administration of the at least one butyrate-producing bacterium. In some cases, a patient can begin metformin therapy at the same time as the patient begins administration of the at least one butyrate-producing bacterium. In some cases, a patient can begin metformin therapy after the patient begins administration of the at least one butyrate-producing bacterium. The combination for use as taught herein may also be administered as a composition as disclosed herein.

The sorbitol or sorbitol source as taught in the present disclosure may be comprised in a formulation in the range of from 55 µmol ([mu]mol) to 550 mmol, preferably from 550 µmol to 280 mmol, more preferably from 2.8 mmol to 55 mmol, most preferably 5.5 mmol to 28 mmol, or for example comprising from 10 mg to 100 g, preferably from 100 mg to 50 g, more preferably from 500 mg to 10 g, most preferably from 1 g to 5 g, e.g. per dose or per ml or per g of composition comprising said. Alternatively, the amount of sorbitol and/or administration frequency is chosen such that it is between 0.01 to 100, preferably from 0.1 to 50, more preferably from 0.5 to 10, most preferably from 1 to 5, all in grams per day.

In an embodiment for use according to the present disclosure, the sorbitol can be formulated in the same composition as the at least one butyrate-producing bacterium, or the sorbitol may be administered separately from the at least one butyrate-producing bacterium. In some cases, a subject can be on sorbitol regimen already when the patient begins administration of the at least one butyrate-producing bacterium. In some cases, a patient can begin sorbitol therapy at the same time as the patient begins administration of the at least one butyrate-producing bacterium. In some cases, a patient can begin sorbitol therapy after the patient begins administration of the at least one butyrate-producing bacterium. The combination for use as taught herein may also be administered as a composition as disclosed herein.

### Composition comprising an insulin-sensitizing and a butyrate-producing bacterium

One of the embodiments of current disclosure comprises the insulin-sensitizing agent and the butyrate-producing bacterium together in a composition. The composition may be in the form of a food composition, feed composition, feed supplement composition, food supplement composition and/or pharmaceutical composition.

One of the embodiments of the composition as taught herein comprises a pharmaceutical composition, preferably in solid dosage form, such as a capsule, a tablet, or a powder. In some embodiments, the pill, tablet or capsule composition comprises an enteric coating designed to release the contents of the pill or capsule composition in an ileum of the subject, a colon of the subject, or a combination thereof. In some embodiments, the composition pill or capsule comprises ingredients for extended or controlled release of the butyrate-producing bacterium and/or insulin-sensitizing agent.

One of the embodiments of the composition as taught herein comprises a food composition, such as a dairy product or a fermented dairy product, preferably a yogurt or a yogurt drink. The food or food supplement composition may be selected from the group consisting of a liquid, liquid beverage (including dairy beverage and fermented beverage), yogurt, cheese, gel, gelatine, gelatine capsule, powder, paste, pressed tablet, and gel cap. In a suitable embodiment, the composition is a liquid, preferably a liquid beverage (e.g. dairy beverage). The food or food supplement composition may be a dairy product, preferably a fermented dairy product, preferably a yogurt or a yogurt drink.

In an embodiment, the butyrate-producing bacterium as taught in the composition is present in an amount ranging from 10⁴ to 10¹⁵ colony forming units (CFU). For instance, the butyrate-producing bacterium may be comprised in the composition in an amount of 10⁶ CFU to 10¹³ CFU, preferably 10⁷ CFU to 10¹² CFU, preferably 10⁸ CFU to 10¹¹ CFU, more preferably 10⁹ CFU to 10¹¹ CFU, e.g. per dose or per ml or per g of composition comprising said. The amount of butyrate-producing bacterium in the composition may also be expressed as the amount per total solid dosage form, e.g. pill, tablet or capsule. Accordingly, the butyrate-producing bacterium as taught herein may be comprised in the composition in an amount ranging from 5x10⁴ to 5x10¹⁵ CFU/g. For instance, the butyrate-producing bacterium may be comprised in the composition in an amount of 5x10⁸ CFU/g to 5x 10¹³ CFU/g, preferably 5x10⁷ CFU/g to 5x10¹² CFU/g, preferably 5x10⁸ CFU/g to 5x10¹¹ CFU/g, more preferably 5x10⁹ CFU/g to 5x10¹¹ CFU/g. Alternatively, the amount of butyrate-producing bacterium in the composition may be expressed as the amount per liquid dosage form, e.g. liquid beverage such as a yogurt or a yogurt drink. Accordingly, the butyrate-producing bacterium as taught herein may be comprised in the composition in an amount ranging from 10² to 10¹³ CFU/ml. For instance, the butyrate-producing bacterium may be comprised in the composition in an amount of 10⁴ CFU/ml to 10¹¹ CFU/ml, preferably 10⁵ CFU/ml to 10¹⁰ CFU/ml, preferably 10⁶ CFU/ml to 10⁹ CFU/ml, more preferably 10⁷ CFU/ml to 10⁹ CFU/ml.

In an embodiment, the inositol as taught in the composition may be present in an amount ranging from 5.5 µmol ([mu]mol) to 5.5 mmol, preferably from 55 µmol to 2.8 mmol, and more preferably from 83 µmol to 278 µmol, or for example comprising from 10 mg to 100 g, preferably from 100 mg to 50 g, more preferably from 500 mg to 10 g, most preferably from 1 g to 5 g. The amount inositol may also be expressed as the amount per total solid dosage form, e.g. pill, tablet or capsule. Accordingly the inositol as taught in the composition may be present in an amount ranging from 275 µmol ([mu]mol)/g to 2.8 mol/g, preferably from 2.8 mmol/g to 1.4 mol/g, more preferably from 14 mmol/g to 275 mmol/g, most preferably 28 mmol/g to 140 mmol/g, or for example comprising from 50 mg to 500 g per solid dosage form, preferably from 500 mg to 250 g solid dosage form, more preferably from 2.5 g to 50 g per g solid dosage form, most preferably from 5 g to 25 g per g solid dosage form. Alternatively, the amount inositol in the composition may be expressed as the amount per liquid dosage form, e.g. liquid beverage such as a yogurt or a yogurt drink. Accordingly, the inositol may be comprised in the combination in the range of from 550 µM ([mu]M) to 5.5 M, preferably from 5.5 mM to 2.8 M, more preferably from 28 mM to 550 mM, most preferably from 55 mM to 280 mM. or for example comprising from 100 mg/L to 1000 g/L, preferably from 1 g/L to 500 g/L, more preferably from 5 g/L to 100 g/L, most preferably from 10 g/L to 50 g/L.

In an embodiment, the chromium as taught in the composition is present in an amount ranging from 24 nmol to 12 µmol ([mu]mol), more preferably from 120 nmol to 5 µmol, and most preferably from 500 nmol to 2.4 µmol, or for example comprising from 10 µg ([mu]g) to 5000 µg, more preferably from 50 µg to 2000 µg, and most preferably from 200 µg to 1000 µg. The amount of chromium may also be expressed as the amount per total solid dosage form, e.g. pill, tablet or capsule. Accordingly, the chromium as taught in the composition may be present in an amount ranging from 120 nmol/g to 60 µmol ([mu]mol)/g, more preferably from 600 nmol/g to 25 µmol/g, and most preferably from 2500 nmol/g to 12 µmol/g, or for example comprising from 50 µg to 25 mg per g solid dosage form, more preferably from 250 µg to 10 mg per g solid dosage form, and most preferably from 1 mg to 5 mg per g solid dosage form. Alternatively, the amount of chromium in the composition may be expressed as the amount per liquid dosage form, e.g. liquid beverage such as a yogurt or a yogurt drink. Accordingly, the chromium may be comprised in the combination in the range of 240 nM to 120 µM ([mu]M), more preferably from 1200 nM to 50 µM, and most preferably from 5000 nM to 24 µM, or for example comprising from 100 µg/L ([mu]g/L) to 50 mg/L, more preferably from 500 µg/L to 20 mg/L, and most preferably from 2 mg/L to 10 mg/L.

In an embodiment, the metformin as taught in the composition is present in an amount ranging from 0.6 mmol to 30 mmol, more preferably from 1.5 mmol to 25 mmol, and most preferably from 6 mmol to 18 mmol, or for example comprising from 100 mg to 5000 mg, more preferably from 250 mg to 4000 mg, and most preferably from 1000 mg to 3000 mg. The amount of metformin may also be expressed as the amount per total solid dosage form, e.g. pill, tablet or capsule. Accordingly, the metformin as taught in the composition may be present in an amount ranging from 3 mmol/g to 150 mmol/g, more preferably from 7.5 mmol/g to 125 mmol/g, and most preferably from 30 mmol/g to 90 mmol/g, or for example comprising from 500 mg to 25 g per g solid dosage form, more preferably from 1.25 g to 20 g per g solid dosage form, and most preferably from 5 g to 15 g per g solid dosage form. Alternatively, the amount of metformin in the composition may be expressed as the amount per liquid dosage form, e.g. liquid beverage such as a yogurt or a yogurt drink. Accordingly, the metformin may be comprised in the combination in the range of 0.6 mM to 300 mM, more preferably from 15 mM to 250 mM, and most preferably from 60 mM to 180 mM, or for example comprising from 1 g/L to 50 g/L, more preferably from 2.5 g/L to 40 g/L, and most preferably from 10 g/L to 30 g/L.

In an embodiment, the sorbitol as taught in the composition may be present in an amount ranging from 5.5 µmol ([mu]mol) to 5.5 mmol, preferably from 55 µmol to 2.8 mmol, and more preferably from 83 µmol to 278 µmol, or for example comprising from 10 mg to 100 g, preferably from 100 mg to 50 g, more preferably from 500 mg to 10 g, most preferably from 1 g to 5 g. The amount of sorbitol may also be expressed as the amount per total solid dosage form, e.g. pill, tablet or capsule. Accordingly the sorbitol as taught in the composition may be present in an amount ranging from 275 µmol ([mu]mol)/g to 2.8 mol/g, preferably from 2.8 mmol/g to 1.4 mol/g, more preferably from 14 mmol/g to 275 mmol/g, most preferably 28 mmol/g to 140 mmol/g, or for example comprising from 50 mg to 500 g per solid dosage form, preferably from 500 mg to 250 g solid dosage form, more preferably from 2.5 g to 50 g per g solid dosage form, most preferably from 5 g to 25 g per g solid dosage form. Alternatively, the amount of sorbitol in the composition may be expressed as the amount per liquid dosage form, e.g. liquid beverage such as a yogurt or a yogurt drink. Accordingly, the sorbitol may be comprised in the combination in the range of from 550 µM ([mu]M) to 5.5 M, preferably from 5.5 mM to 2.8 M, more preferably from 28 mM to 550 mM, most preferably from 55 mM to 280 mM. or for example comprising from 100 mg/L to 1000 g/L, preferably from 1 g/L to 500 g/L, more preferably from 5 g/L to 100 g/L, most preferably from 10 g/L to 50 g/L.

One of the embodiments of the composition as taught herein comprises a physiologically acceptable carrier, wherein the carrier suitable for keeping the butyrate-producing bacterium as taught herein viable until consumption by a subject (e.g. human or animal). For instance, non-limiting examples of acceptable carriers that are suitable for this purpose include any of well-known physiological or pharmaceutical carriers, buffers, and excipients. It will be appreciated that the choice for a suitable physiological or pharmaceutical carrier will depend upon the intended mode of administration of the composition as taught herein (e.g. oral) and the intended form of the composition (e.g. beverage, yogurt, powder, capsules, and the like). The skilled person knows how to select a physiological or pharmaceutical carrier, which is suitable for the compositions as taught herein. Preferably, a pharmaceutical carrier does not relate to pure water or aqueous medium comprising more than 99 wt.% water.

The composition as taught herein may be a pharmaceutical composition. The pharmaceutical composition may be for use as a supplement. A pharmaceutical composition will usually comprise a pharmaceutical carrier, in addition to the at least one bacterium and at least one insulin-sensitizing agent as taught herein. The carrier is preferably an inert carrier. The preferred form depends on the intended mode of administration and (therapeutic) application. A pharmaceutical carrier can be any compatible, nontoxic substance suitable to deliver the bacterium taught herein to the GI tract of a subject. For example, sterile water, or inert solids complemented with a pharmaceutically acceptable adjuvant, buffering agent, dispersing agent, and the like. A pharmaceutical composition as taught herein may be in liquid form, e.g. a stabilized suspension of bacteria of the bacterium taught herein, or in solid form, e.g., a powder of lyophilized bacteria taught herein. In case the bacterium taught herein is lyophilized, a cryoprotectant such as lactose, trehalose or glycogen can be employed. E.g., for oral administration, the bacterium taught herein can be administered in solid dosage forms, such as capsules, tablets, and powders, comprising lyophilized bacteria, or in liquid dosage forms, such as elixirs, syrups, and suspensions. The bacterium taught herein, e.g., in lyophilized form, can be encapsulated in capsules such as gelatin capsules, together with inactive ingredients and powdered carriers, such as e.g. glucose, lactose, sucrose, mannitol, starch, cellulose or cellulose derivatives, magnesium stearate, stearic acid, sodium saccharin, talcum, magnesium carbonate and the like.

In one embodiment, the compositions as taught herein may further comprise one or more ingredients, which further enhance the nutritional value and/or the therapeutic value the compositions as taught herein. For instance, it may be advantageous to add one or more ingredients (e.g. nutritional ingredients, veterinary or medicinal agents etc.) selected from proteins, amino acids, enzymes, mineral salts, vitamins (e.g. thiamine HCl, riboflavin, pyridoxine HCl, niacin, choline chloride, calcium pantothenate, biotin, folic acid, ascorbic acid, vitamin B12, p-aminobenzoic acid, vitamin A acetate, vitamin K, vitamin D, vitamin E, and the like), sugars and complex carbohydrates (e.g. water-soluble and water-insoluble monosaccharides, disaccharides, and polysaccharides), medicinal compounds (e.g. antibiotics), antioxidants, trace element ingredients (e.g. compounds of cobalt, copper, manganese, iron, zinc, tin, nickel, molybdenum, iodine, chlorine, silicon, vanadium, selenium, calcium, magnesium, sodium and potassium and the like). The skilled person is familiar with methods and ingredients that are suitable to enhance the nutritional and/or therapeutic/medicinal value of the compositions as taught herein.

### PREFERRED EMBODIMENTS

Examples of preferred embodiments as taught in the present disclosure may include or exclude the combination of *Anaerobutyricum soehngenii* or relative thereof having a 16S rRNA gene sequence with at least 97% sequence identity with SEQ ID NO:1 or SEQ ID NO:2 with:
- chromium;
- chromium (III) picolinate;
- inositol;
- myo-inositol;
- metformin;
- metformin hydrochloride; and/or
- sorbitol.

Other examples of preferred embodiments as taught in the present disclosure may include or exclude the combination of *Intestinimonas butyriciproducens* or relative thereof having a 16S rRNA gene sequence with at least 97% sequence identity with SEQ ID NO:3 with:
- chromium;
- chromium (III) picolinate;
- inositol;
- myo-inositol;
- metformin;
- metformin hydrochloride; and/or
- sorbitol.

Yet other examples of preferred embodiments as taught in the present disclosure may include or exclude the combination of *Anaerostipes rhamnosivorans* or relative thereof having a 16S rRNA gene sequence with at least 97% sequence identity with SEQ ID NO:4 with:
- chromium;
- chromium (III) picolinate;
- inositol;
- myo-inositol;
- metformin;
- metformin hydrochloride; and/or
- sorbitol.

A more preferred embodiment as taught in the present disclosure is a composition comprising *Anaerobutyricum soehngenii* or relative thereof having a 16S rRNA gene sequence with at least 97% sequence identity with SEQ ID NO:1 or SEQ ID NO:2 with chromium(III) picolinate, preferably as a dairy product, more preferably a fermented dairy product, preferably a yogurt or a yogurt drink.

Similarly, a more preferred embodiments as taught in the present disclosure is a composition comprising *Anaerobutyricum soehngenii* or relative thereof having a 16S rRNA gene sequence with at least 97% sequence identity with SEQ ID NO:1 or SEQ ID NO:2 with chromium(III) picolinate, preferably in a solid dosage form, such as a capsule, a tablet, or a powder.

Also, a preferred embodiment as taught in the present disclosure is a composition comprising *Anaerobutyricum soehngenii* or relative thereof having a 16S rRNA gene sequence with at least 97% sequence identity with SEQ ID NO:1 or SEQ ID NO:2 with sorbitol, preferably as a dairy product, more preferably a fermented dairy product, preferably a yogurt or a yogurt drink, or as a solid dosage form, such as a capsule, a tablet, or a powder.

Hence, in the present disclosure, if the insulin-sensitizing agent is sorbitol, the butyrate-producing bacterium preferably is *Anaerobutyricum soehngenii* or relative thereof having a 16S rRNA gene sequence with at least 97% sequence identity with SEQ ID NO:1 or SEQ ID NO:2.

Examples of preferred embodiments as taught in the present disclosure may include or exclude the combination of micro-encapsulated butyrate-producing bacteria with insulin-sensitizing agents.

Examples of a preferred embodiment as taught in the present disclosure may include or exclude the combination of micro-encapsulated *Anaerobutyricum soehngenii* or relative thereof having a 16S rRNA gene sequence with at least 97% sequence identity with SEQ ID NO:1 or SEQ ID NO:2 with:
- chromium;
- chromium (III) picolinate;
- inositol;
- myo-inositol;
- metformin;
- metformin hydrochloride; and/or
- sorbitol.

Other examples of preferred embodiments as taught in the present disclosure may include or exclude the combination of micro-encapsulated *Intestinimonas butyriciproducens* or relative thereof having a 16S rRNA gene sequence with at least 97% sequence identity with SEQ ID NO:3 with:
- chromium;
- chromium (III) picolinate;
- inositol;
- myo-inositol;
- metformin;
- metformin hydrochloride; and/or
- sorbitol.

Yet other examples of preferred embodiments as taught in the present disclosure may include or exclude the combination of micro-encapsulated *Anaerostipes rhamnosivorans* or relative thereof having a 16S rRNA gene sequence with at least 97% sequence identity with SEQ ID NO:4 with:
- chromium;
- chromium (III) picolinate;
- inositol;
- myo-inositol;
- metformin;
- metformin hydrochloride; and/or
- sorbitol.

A preferred embodiment as taught in the present disclosure is a composition comprising micro-encapsulated *Anaerobutyricum soehngenii* or relative thereof having a 16S rRNA gene sequence with at least 97% sequence identity with SEQ ID NO:1 or SEQ ID NO:2 with chromium(III) picolinate, preferably in a solid dosage form, such as a capsule, a tablet, or a powder.

Another preferred embodiment as taught in the present disclosure is a composition comprising micro-encapsulated *Intestinimonas butyriciproducens or relative thereof having a 16S rRNA gene sequence with at least 97% sequence identity with SEQ ID NO:3* with chromium(III) picolinate, preferably in a solid dosage form, such as a capsule, a tablet, or a powder.

Yet another preferred embodiment as taught in the present disclosure is a composition comprising micro-encapsulated *Anaerostipes rhamnosivorans* or relative thereof having a 16S rRNA gene sequence with at least 97% sequence identity with SEQ ID NO:4 with chromium(III) picolinate, preferably in a solid dosage form, such as a capsule, a tablet, or a powder.

Optionally, the butyrate-producing bacterium is micro-encapsulated (or lyophilized) in case one of the following provisos apply in full
- the insulin-sensitizing agent is metformin and the butyrate-producing bacterium is *Anaerobutyricum soehngenii* or a relative thereof having a 16S rRNA gene sequence with at least 97% sequence identity with SEQ ID NO:1 or SEQ ID NO:2; or
- the insulin-sensitizing agent is chromium or inositol and the butyrate-producing bacterium is *Intestinimonas butyriciproducens* or relative thereof having a 16S rRNA gene sequence with at least 97% sequence identity with SEQ ID NO:3 or *Anaerostipes rhamnosivorans* or relative thereof having a 16S rRNA gene sequence with at least 97% sequence identity with SEQ ID NO:4.

The combination according to the present disclosure may be used for preventing and/or treating metabolic disease, insulin resistance and/or insulin resistance-related conditions as set out herein, and/or the combination according to the present disclosure may be used for (enhancing) butyric acid and/or butyrate production, preferably *in situ,* i.e. in the small intestine.

In addition or alternatively, the present disclosure may exclude prevention and/or treatment of vitamin B12 deficiency.

The terms 'comprising' or 'to comprise' and their conjugations, as used herein, refer to a situation wherein said terms are used in their non-limiting sense to mean that items following the word are included, but items not specifically mentioned are not excluded. It also encompasses the more limiting verb 'to consist essentially of' and 'to consist of'.

Reference to an element by the indefinite article 'a' or 'an' does not exclude the possibility that more than one of the elements is present, unless the context clearly requires that there be one and only one of the elements. The indefinite article 'a' or 'an' thus usually means 'at least one'.

The terms 'to increase' and 'increased level' and the terms 'to decrease' and 'decreased level' refer to the ability to significantly increase or significantly decrease or to a significantly increased level or significantly decreased level. Generally, a level is increased or decreased when it is at least 5%, such as 10%, 15%, 20%, 25%, 30%, 35%, 40%, 45%, 50% higher or lower, respectively, than the corresponding level in a control or reference. Alternatively, a level in a sample may be increased or decreased when it is statistically significantly increased or decreased compared to a level in a control or reference.

Should there be an inconsistency between the sequences disclosed in the description and the sequences disclosed in the sequence listing, the sequences disclosed in the description are preferred. Alternatively, the sequences of the sequence listing may be used.

As used herein, the term "identity" refers to a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A. M., ed., Oxford University Press, New York, 1988; BIOCOMPUTING: INFORMATICS AND GENOME PROJECTS, Smith, D. W., ed., Academic Press, New York, 1993; COMPUTER ANALYSIS OF SEQUENCE DATA, PART I, Griffin, A. M., and Griffin, H. G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER; Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., SIAM J. Applied Math (1988) 48:1073). Methods commonly employed to determine identity or similarity between two sequences include, but are not limited to, those disclosed in GUIDE TO HUGE COMPUTERS, Martin J. Bishop, ed., Academic Press, San Diego, 1994, and Carillo, H., and Lipton, D., SIAM J. Applied Math (1988) 48:1073. Methods to determine identity and similarity are codified in computer programs. For example NCBI Nucletide Blast with standard settings (blastn, https://blast.ncbi.nlm.nih.gov/). Preferred computer program methods to determine identity and similarity between two sequences include, but are not limited to, GCS program package (Devereux, J., et al., Nucleic Acids Research (1984) 12(1):387), BLASTP, BLASTN, FASTA (Atschul, S. F. et al., J. Molec. Biol. (1990) 215:403). As an illustration, by a nucleotide sequence having at least, for example, 95% "identity" to a reference nucleotide sequence, it is intended that the nucleotide sequence is identical to the reference sequence except that there may be up to five point mutations per each 100 nucleotides of the reference polypeptide sequence. In other words, to obtain a nucleotide sequence being at least 95% identical to a reference nucleotide sequence, up to 5% of the nucleotides in the reference sequence may be deleted and/or substituted with another nucleotide, and/or a number of nucleotides up to 5% of the total nucleotides in the reference sequence may be inserted into the reference sequence. Preferably, the sequence identity refers to the sequence identity over the entire length of the sequence. It is further understood that, when referring to "sequences" herein, generally the actual physical molecules with a certain sequence of subunits (e.g. amino acids) are referred to.

### Brief description of the Figure

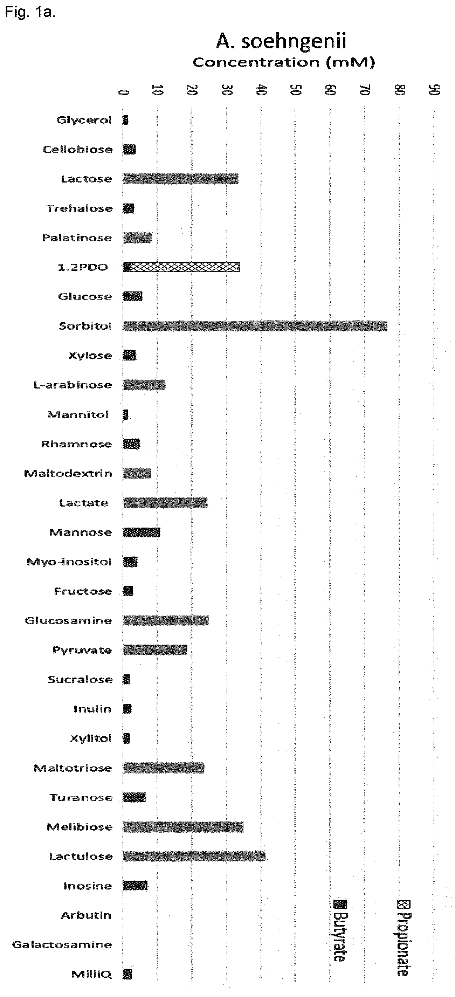

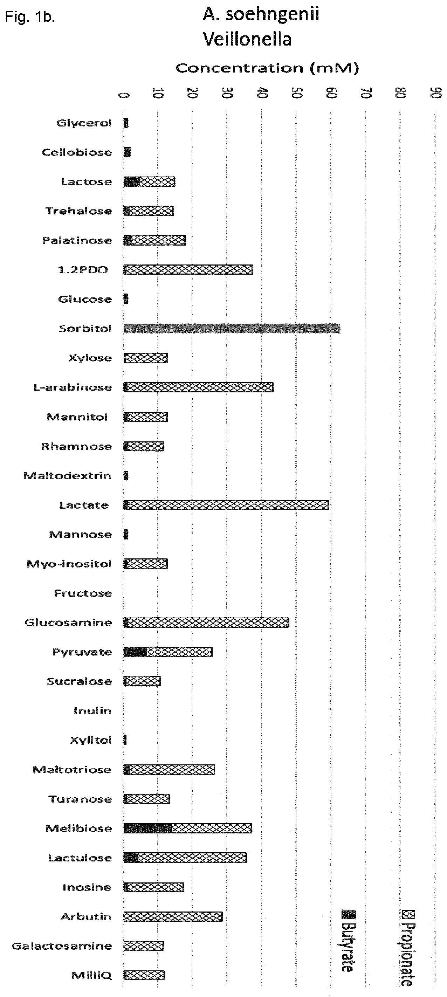

Figure 1 (a, b): The ability of *Anaerobutyricum soehngenii* to produce butyrate was tested (a) in the presence of different substrates and (b) in the presence of *Veillonella spp.* The concentration of butyrate and propionate is provided in mM.

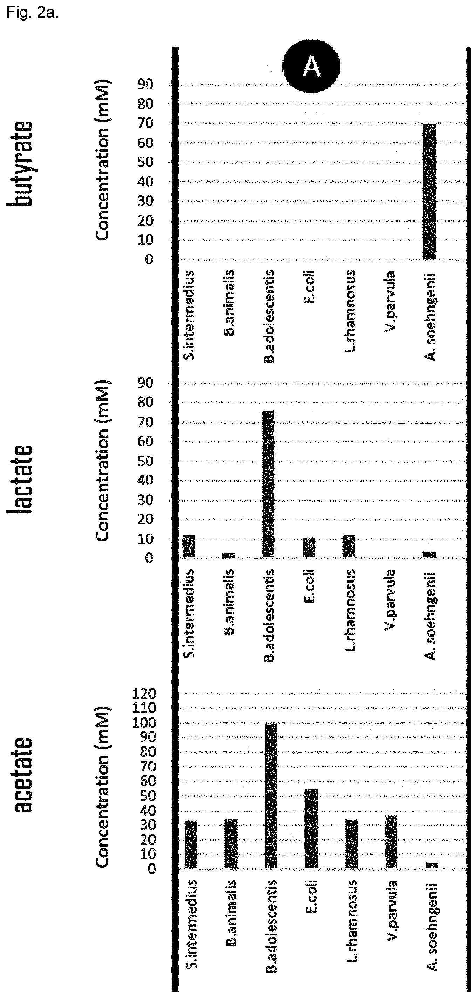

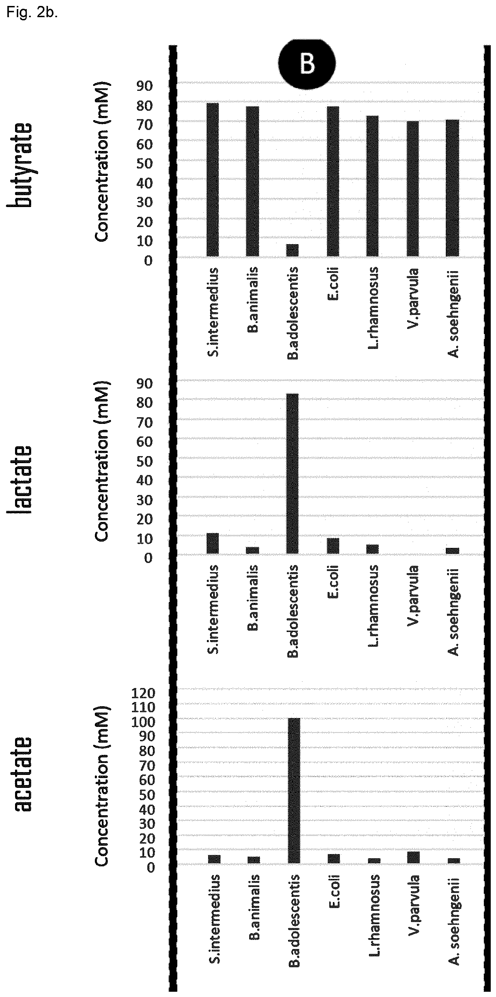

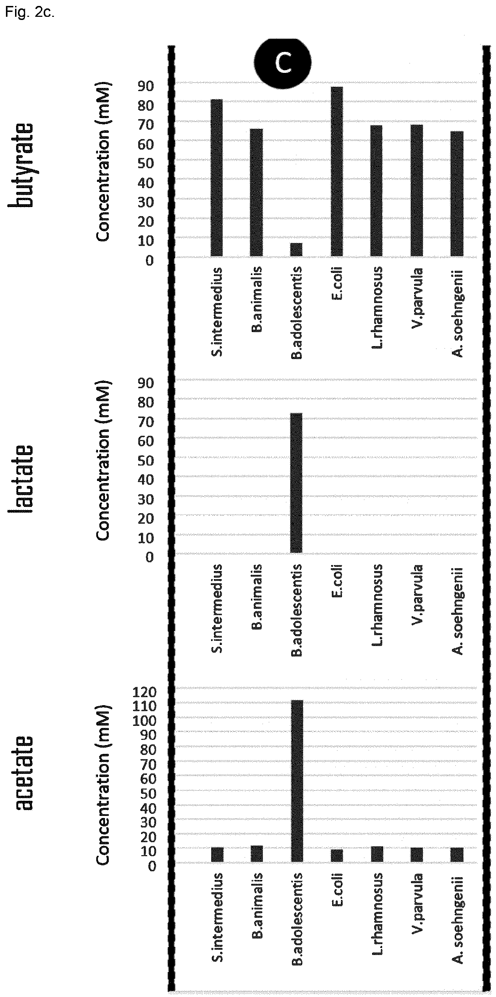

Figure 2 (a, b, c): It was tested whether representative bacterial species as present in the small intestine are able to metabolize sorbitol. (a=sorbitol + indicated strain; b=sorbitol + indicated strain + *A. soehngenii;* c=sorbitol + indicated strain + *A. soehngenii + Veilonella parvula*)*.* The species tested included *S. intermedius, B. animalis, B. adolescentis, E. coli, L. rhamnosus, V. parvula, A. soehngenii.* From the tested species, apart from *A. soehngenii,* only *B*. *adolescentis* was able to metabolize sorbitol, and produced lactate and acetate. The concentration of butyrate, lactate and acetate is provided in mM.

### EXPERIMENTAL EXAMPLE 1

### (Non-)insulin-sensitizing agents and (non-)butyrate-producing bacteria

It is known that insulin-sensitizing agents can reduce insulin resistance. The present inventors consider that co-administration of insulin-sensitizing agents and butyrate-producing bacteria may have a beneficial and synergistic effect on preventing or treating metabolic disease, insulin resistance or insulin resistance-related conditions.

As shown in this experiment, the present inventors determine the therapeutic effect of insulin-sensitizing agents when administered alone, when administered together with butyrate-producing bacteria, or when administered together with a non-butyrate-producing bacterium.

Myo-inositol, chromium(III) picolinate, metformin, and sorbitol are examples of insulin-sensitizing agents according to the current disclosure. Ascorbic acid and calcium are examples of non-insulin-sensitizing agents.

Ascorbic acid is an example of the group of vitamin C. Ascorbic acid is involved in biological oxidation- and reduction processes. Ascorbic acid also plays a role in the synthesis of hormones, collagen, and in hematopoiesis. Ascorbic acid is mostly used for the treatment of scurvy or in conditions where increased vitamin C levels are beneficial. The exact mechanism of the therapeutic effect is not entirely known. Ascorbic acid is used herein as an example of a non-insulin-sensitizing agent.

Calcium is involved in the formation and remodeling of the skeleton. Calcium furthermore is involved in regulation of various biological processes such as nerve and muscle function, hormone action, blood coagulation, and cell motility. Calcium, along with vitamin D, may possibly protect against cancer, diabetes and high blood pressure, although the evidence is not definitive. Calcium is used for the prevention/treatment of calcium deficiency or as a supplement in the treatment of osteoporosis. Calcium is used herein as a second example of a non-insulin-sensitizing-agent.

*A. soehngenii, A. rhamnosirovans,* and *I. butyriciproducens* are examples of butyrate-producing bacteria.

*Bifidobacterium animalis* subsp. *Lactis* is an example of a non-butyrate-producing bacterium. *B. animalis* is present in many food products and dietary supplements. The probiotic is mostly found in dairy products. *Bifidobacterium animalis* subsp. *Lactis* belongs to the phylum of *Actinobacteria* and like all Bifidobacteria is producing mainly lactate and acetate as end products during growth.

### Treatment

Mixed male/female subjects aged 18-65 years with a HOMA(-IR) score between 2.5 and 3.5, thus indicating insulin resistance, are included in the study. The HOMA-IR can be determined as described herein elsewhere. Subjects are treated for 28 days according to the single or combinatorial treatment arms shown in Table 1. The HOMA(-IR) is measured at baseline and after 28 days. A reduction in HOMA(-IR) after treatment indicates a relative decrease in insulin resistance. The efficacy in reduction of the HOMA(-IR) following treatment is shown in Table 1 accordingly to the following ranking system, wherein the first rank describes the lowest effect and the last rank describes the highest effect: 'non-measurable', 'very low, 'low', 'low/medium', 'medium', 'high', 'very high'.

Microbiota treatment is given in capsule form, at 10¹⁰ living units per capsule, once daily. Insulin-sensitizing agents are given in capsule form, according to the following scheme: metformin hydrochloride, 500 mg three times daily; myo-inositol, 2000 mg once daily; chromium picolinate, 500 mcg once daily. Non-insulin-sensitizing agents are given in capsule form, according to the following scheme: ascorbic acid, 500 mg once daily; calcium, 500 mg twice daily.

### Administration of (non-)butyrate-producing bacteria in combination with (non-)insulin-sensitizing agents

The insulin-sensitizing agents alone have limited ability to improve insulin sensitivity. However, when combined with the butyrate-producing bacterium, the insulin-sensitizing agents enhance insulin sensitivity through a synergistic action together with the butyrate-producing bacterium. Similarly, the non-butyrate producing strains have modest ability to improve insulin sensitivity, but which is further enhanced through a synergistic action together with insulin-sensitizing agents (Table 1). Even more surprisingly, the combination of *A. soehngenii* (or *A. hallii)* with chromium(lll) picolinate leads to an even further increased synergistic improvement of insulin sensitivity and the highest insulin-sensitizing effect.

This example is used to illustrate that at least one butyrate-producing bacterial strain and at least one insulin-sensitizing agent have a synergistic effect in reducing insulin resistance. At the same time, this example is used to illustrate that the combination of *A. soehngenii* (or related strains such as *A. hallii*), together with chromium or alternate forms of chromium may lead to the largest therapeutic effect in terms of decreasing insulin resistance and therefore may be a preferred embodiment of present disclosure for prevention/treatment of metabolic diseases, such as insulin resistance and/or insulin resistance-related conditions.

**Table 1: treatment scheme used to show the putative reduction in insulin resistance (3 recipients per condition) as measured by the reduction in HOMA-IR value.**

| **Supplement Bacterium** | **Placebo** | **Chromium(III) picolinate** | **Myo-inositol** | **Metformin** | **Sorbitol** | **Ascorbic acid** | **Calcium** |
|---|---|---|---|---|---|---|---|
| | | **Insulin-sensitizing** | | | | **Non-insulin-sensitizing** | |
| **Placebo** | Non-measurable | Very low | Very low | Low/medium | Low/medium | Non-measurable | Non-measurable |
| ***A. soehngenii* (butyrate producing)** | Medium | Very high | High | High | High | Medium | Medium |
| ***A. rhamnosirovans* (butyrate producing)** | Medium | High | High | High | High | Medium | Medium |
| ***I. butyriciproducens* (butyrate producing)** | Medium | High | High | High | High | Medium | Medium |
| ***Bifidobacterium animalis* subsp. *lactis* (non-butyrate producing)** | Non-measurable | Very low | Very low | Very low | Very low | Non-measurable | Non-measurable |

It is expected that results similar to the putative effects as shown in Table 1 can be obtained with larger patient cohorts. It is also expected that the effects as measured by changes in HOMA-IR, are also measurable for a more extensive list of non-butyrate-producing strains or non-insulin sensitizing agents.

Moreover, it is expected that similar improvements as listed in Table 1 are alternatively observed for the fasting serum insulin level. A fasting serum level greater than 25 mU/L has been considered as indicating insulin resistance. The skilled person in the art knows the most-widely accepted and/or most accurate ways of measuring the fasting serum levels, and is generally measured after at least 8 hours fasting after the last meal. The measurement of fasting serum level is generally performed as part of measuring HOMA(-IR), as described herein elsewhere.

It is further expected that similar improvements as listed in Table 1 are alternatively observed for the Glycated haemoglobin (HbA1c). Circulating blood glucose attaches to haemoglobin and concentrations of the resulting glycated haemoglobin, or 'HbA1c', reflect levels of blood glucose in the preceding 8-12 weeks. The skilled person in the art knows the most-widely accepted and/or most accurate ways of measuring HbA1c. The main analytical methods used for the measurement of HbA1c include affinity chromatography, immunoassay, cation exchange chromatography, and capillary electrophoresis.

### Synergistic effect on lowering insulin resistance

The supplementation of a subject_with a butyrate-producing bacterium and an insulin-sensitizing agent according to the present disclosure provides an unexpected, synergistic effect with respect to reducing insulin resistance. In particular, the supplementation of a subject with *A. soehngenii* and chromium according to the present disclosure, provides a surprisingly large synergistic effect. Such a synergistic interaction between insulin-sensitizing agents and butyrate-producing bacteria has not been reported to date. The co-administration of an insulin-sensitizing agent and a butyrate-producing bacterium is believed to be a highly efficient way of preventing and/or treating metabolic diseases, such as insulin resistance and insulin resistance-related conditions, preferably chosen from type 1 diabetes mellitus , type 2 diabetes mellitus, dyslipidemia, metabolic syndrome, insulin resistance in endocrine disease, Polycystic Ovary Syndrome (PCOS), Nonalcoholic fatty liver disease (NAFLD), and nonalcoholic steatohepatitis (NASH).

The exact mechanism by which insulin-sensitizing agents and butyrate-producing bacteria synergistically decrease insulin resistance, among many other possible effects, is unclear. It cannot be excluded that that the production of butyrate by the bacterial strains, in part, may synergistically improve the effect of insulin-sensitizing agents. Alternatively, it cannot be excluded that the insulin-sensitizing agents, in part, may also further enhance the butyrate production by the bacteria. Without being bound by theory, both of these possible mechanisms follow the premise that the synergistic effect on reducing insulin resistance was not observed in the combinations comprising *Bifidobacterium animalis* subsp. *lactis,* as can be seen above. Butyrate has been suggested to act on food intake through G-protein-coupled receptors such as GPR41 and GPR43, which subsequently increase release of the satiety hormones PYY and GLP-1. Furthermore, butyrate has been implicated in regulation of intestinal gluconeogenesis thereby improving glucose and energy homeostasis (Udayappan et al. NPJ Biofilms Microbiomes. 2016 Jul 6;2:16009). Butyrate may also regulate the activity of Peroxisome proliferator-activated receptor (PPAR)-y coactivator (PGC)-1α, a transcription coactivator involved in mitochondrial function (Gao et al. Diabetes. 2009 Jul; 58(7): 1509-1517).

### EXPERIMENTAL EXAMPLE 2

### Micro-encapsulation of butyrate-producing bacteria

Micro-encapsulation of butyrate-producing bacteria improves their insulin-sensitizing effect in combination with insulin-sensitizing agents.

As shown in this experiment, the present inventors compare the insulin-sensitizing effect of non-micro-encapsulated butyrate-producing bacteria in combination with insulin-sensitizing agents and micro-encapsulated butyrate-producing bacteria in combination with insulin-sensitizing agents.

Without being bound to a single method for the micro-encapsulation of butyrate-producing bacteria, alginate-chitosan micro-encapsulation is used herein to illustrate the effect of micro-encapsulation on the insulin-sensitizing effect of butyrate-producing bacteria in combination with insulin-sensitizing agents. *A. soehngenii, A. rhamnosirovans,* and *I. butyriciproducens* are used as butyrate-producing bacteria. Chromium, (myo)inositol, metformin, and sorbitol are used as insulin-sensitizing agent.

### Treatment

The same inclusion criteria of subjects and measurements of HOMA(-IR) are used as described in experimental example 1. The same ranking system is used as described in experimental example 1 to show the efficacy in the reduction in HOMA(-IR). The applied dose of butyrate-producing bacteria is 100-fold lower as compared to experimental example 1 to exemplify the effect of bacterial micro-encapsulation on the insulin-sensitizing effect in combination with insulin-sensitizing agents.

The bacteria are given in capsule form, at 10⁸ living units per capsule once daily. Chromium picolinate is given in capsule form, at 500 mcg once daily.

In brief, alginate-chitosan micro-encapsulation of butyrate-producing bacteria involves mixing the bacterial suspension into a polymeric alginate solution. Alginate beads are gelated using calcium chloride and subsequently submerged in a chitosan solution to provide a secondary coating. The micro-encapsulated butyrate-producing bacteria are subsequently further processed to provide the capsule form.

### Administration of (non-)micro-encapsulated bacteria in combination with insulin-sensitizing agents

**Table 2: treatment scheme used to show the putative reduction in insulin resistance (3 recipients per condition) as measured by the reduction in HOMA-IR value.**

| **Supplement Bacterium** | **Placebo** | **Insulin-sensitizing agent** |
|---|---|---|
| **Placebo** | Non-measurable | Very low |
| ***A. soehngenii*** | Low | High |
| ***A. soehngenii* micro-encapsulated** | Low/medium | Very high |
| ***A. rhamnosirovans*** | Low | Medium |
| ***A. rhamnosirovans* micro-encapsulated** | Low/medium | Very high |
| ***I. butyriciproducens*** | Low | Medium |
| ***I. butyriciproducens* micro-encapsulated** | Low/medium | Very high |

Table 2 exemplifies that the insulin-sensitizing effect of butyrate-producing bacteria in combination with an insulin-sensitizing agent is largest when the butyrate-producing bacteria are micro-encapsulated. Although the butyrate-producing bacteria and insulin-sensitizing agent provide a synergistic effect with respect to reducing insulin resistance, this therapeutic effect is greatest when the butyrate-producing bacteria are micro-encapsulated.

It is expected that similar effects as shown in Table 2 are also obtained with larger patient cohorts. It is also expected that the effects as measured by changes in HOMA-IR, are also measurable for a more extensive list of non-butyrate-producing strains or non-insulin sensitizing agents. Moreover, it is expected that similar improvements as listed in Table 2 are alternatively observed for the fasting serum insulin level. It is expected that similar improvements as listed in Table 2 are alternatively observed for the glycated haemoglobin (HbA1c).

The present inventors expect that other micro-encapsulation methods may also lead to a further improvement of the insulin-sensitizing effect of butyrate-producing bacteria in combination with insulin-sensitizing agents.

This example is used to illustrate that the combination of micro-encapsulated butyrate-producing bacteria and insulin-sensitizing agents leads to an unexpected higher therapeutic effect and may therefore be a preferred embodiment of the present disclosure for prevention/treatment of metabolic diseases, such as insulin resistance and/or insulin resistance-related conditions.

The exact mechanism by which micro-encapsulated butyrate-producing bacteria and insulin-sensitizing agents have further enhanced synergistic effects in decreasing insulin resistance, among many other possible effects, is unclear.

### EXPERIMENTAL EXAMPLE 3

In this example, the ability of *Anaerobutyricum soehngenii* to produce butyrate was tested (1) in the presence of different substrates and (2) in the presence of other bacteria that colonize the small intestine including *S. intermedius, B. animalis, B. adolescentis, E. coli, L. rhamnosus,* and *V. parvula.*

It was found that *Anaerobutyricum soehngenii* competes for most of the substrates with *Veillonella spp.* (which are naturally present in the small intestine). Accordingly, for most of the substrates, butyrate production by *Anaerobutyricum soehngenii* was found to be limited by the presence of *Veillonella spp.*

Surprisingly, it was found that *Anaerobutyricum soehngenii* does not or to a much lesser extent compete with *Veillonella spp.* or any other bacteria in the small intestine when the substrate is sorbitol, which appears to be a very promising combination with *Anaerobutyricum soehngenii* for enhancing butyrate production *in vivo,* i.e. *in situ* in the small intestine. See Figure 1.

Accordingly, the present inventors consider that providing *Anaerobutyricum soehngenii* together with sorbitol may create a local niche for *Anaerobutyricum soehngenii* leading to easier colonization *in situ* in the small intestine.

Next, it was tested whether representative bacterial species as present in the small intestine are able to metabolize sorbitol. The species tested included *S. intermedius, B. animalis, B. adolescentis, E. coli, L. rhamnosus, V. parvula, A. soehngenii.* From the tested species, apart from *A. soehngenii,* only *B*. *adolescentis* was able to metabolize sorbitol, and produced lactate and acetate. See Figure 2 (a=sorbitol + indicated strain; b=sorbitol + indicated strain + *A. soehngenii;* c=sorbitol + indicated strain + *A. soehngenii + Veilonella parvula).* In Figure 2a, some acetate production is visible by bacteria other than *B*. *adolescentis,* which is due to remaining sugars as present in the medium. As can be seen in Figure 2b, this acetate production disappears in the presence of *A. soehngenii.*

## Claims

1. Insulin-sensitizing agent chosen from the group consisting of
- chromium;
- inositol;
- metformin; and
- sorbitol for use in preventing and/or treating insulin resistance and/or insulin resistance-related condition, wherein the insulin-sensitizing agent is combined with a butyrate-producing bacterium chosen from the group consisting of
- *Anaerobutyricum soehngenii* or relative thereof having a 16S rRNA gene sequence with at least 97% sequence identity with SEQ ID NO:1 or SEQ ID NO:2;
- *Intestinimonas butyriciproducens* or relative thereof having a 16S rRNA gene sequence with at least 97% sequence identity with SEQ ID NO:3; and
- *Anaerostipes rhamnosivorans* or relative thereof having a 16S rRNA gene sequence with at least 97% sequence identity with SEQ ID NO:4,
wherein the butyrate-producing bacterium is micro-encapsulated in case
- the insulin-sensitizing agent is metformin and the butyrate-producing bacterium is *Anaerobutyricum soehngenii* or a relative thereof having a 16S rRNA gene sequence with at least 97% sequence identity with SEQ ID NO:1 or SEQ ID NO:2; or
- the insulin-sensitizing agent is chromium or inositol and the butyrate-producing bacterium is *Intestinimonas butyriciproducens* or relative thereof having a 16S rRNA gene sequence with at least 97% sequence identity with SEQ ID NO:3 or *Anaerostipes rhamnosivorans* or relative thereof having a 16S rRNA gene sequence with at least 97% sequence identity with SEQ ID NO:4,
wherein the insulin resistance-related condition is chosen from the group consisting of metabolic syndrome, type 1 diabetes mellitus, type 2 diabetes mellitus, dyslipidemia, insulin resistance in endocrine disease, Polycystic Ovary Syndrome (PCOS), Nonalcoholic fatty liver disease (NAFLD), and/or nonalcoholic steatohepatitis (NASH).

2. Insulin-sensitizing agent in combination with a butyrate-producing bacterium for use according to claim 1, wherein the butyrate-producing bacterium is micro-encapsulated.

3. Insulin-sensitizing agent in combination with a butyrate-producing bacterium for use according to any one of the preceding claims, wherein the insulin-sensitizing agent is chromium, and the butyrate-producing bacterium is *Anaerobutyricum soehngenii* or relative thereof having a 16S rRNA gene sequence with at least 97% sequence identity with SEQ ID NO:1 or SEQ ID NO:2.

4. Insulin-sensitizing agent in combination with a butyrate-producing bacterium for use according to any one of the preceding claims, wherein the chromium is chromium(III) picolinate.

5. Insulin-sensitizing agent in combination with a butyrate-producing bacterium for use according to any one of the preceding claims, wherein the butyrate-producing bacterium is administered separately, sequentially or simultaneously to the insulin-sensitizing agent.

6. Insulin-sensitizing agent in combination with a butyrate-producing bacterium for use according to any one of the preceding claims, wherein the butyrate-producing bacterium is present in an amount ranging from 10⁴ to 10¹⁵ colony forming units (CFU).

7. Insulin-sensitizing agent in combination with a butyrate-producing bacterium for use according to any one of the preceding claims, wherein the butyrate-producing bacterium is present in lyophilized form.

8. Insulin-sensitizing agent in combination with a butyrate-producing bacterium for use according to any one of the preceding claims, wherein metformin is present in an amount ranging from 100 mg to 5000 mg.

9. Insulin-sensitizing agent in combination with a butyrate-producing bacterium for use according to any one of the preceding claims, wherein chromium is present in an amount ranging from 10 µg to 5000 µg.

10. Insulin-sensitizing agent in combination with a butyrate-producing bacterium for use according to any one of the preceding claims, wherein inositol is present in an amount ranging from 10 mg to 100 g.

11. Insulin-sensitizing agent in combination with a butyrate-producing bacterium for use according any one of the preceding claims, wherein the insulin-sensitizing agent and the butyrate-producing bacterium are comprised in a composition.

12. Insulin-sensitizing agent in combination with a butyrate-producing bacterium for use according to claim 11 wherein the composition comprises a physiologically acceptable carrier.

13. Insulin-sensitizing agent in combination with a butyrate-producing bacterium for use according to any one of claims 11-12, wherein the composition is a pharmaceutical composition, preferably in solid dosage form, such as a capsule, a tablet, or a powder.

14. Insulin-sensitizing agent in combination with a butyrate-producing bacterium for use according to any one of claims 11-12, wherein the composition is a food composition, preferably a dairy product, more preferably a fermented dairy product, most preferably a yogurt or a yogurt drink.

## Patentansprüche

1. Insulinsensibilisierungsmittel, ausgewählt aus der Gruppe bestehend aus
- Chrom;
- Inosit;
- Metformin; und
- Sorbit zur Verwendung bei der Vorbeugung und/oder Behandlung von Insulinresistenz und/oder einem mit Insulinresistenz verbundenen Leiden, wobei das Insulinsensibilisierungsmittel mit einem Butyrat produzierenden Bakterium kombiniert ist, das ausgewählt ist aus der Gruppe bestehend aus
- *Anaerobutyricum soehngenii* oder Verwandten davon mit einer 16S-rRNA-Gensequenz mit einer Sequenzidentität von mindestens 97 % mit SEQ ID NO:1 oder SEQ ID NO:2;
- *Intestinimonas butyriciproducens* oder Verwandten davon mit einer 16S-rRNA-Gensequenz mit einer Sequenzidentität von mindestens 97 % mit SEQ ID NO:3; und
- *Anaerostipes rhamnosivorans* oder Verwandten davon mit einer 16S-rRNA-Gensequenz mit einer Sequenzidentität von mindestens 97 % mit SEQ ID NO:4,
wobei das Butyrat produzierende Bakterium mikroverkapselt ist, falls
- es sich bei dem Insulinsensibilisierungsmittel um Metformin und bei dem Butyrat produzierenden Bakterium um *Anaerobutyricum soehngenii* oder eine Verwandte davon mit einer 16S-rRNA-Gensequenz mit einer Sequenzidentität von mindestens 97 % mit SEQ ID NO:1 oder SEQ ID NO:2 handelt; oder
- es sich bei dem Insulinsensibilisierungsmittel um Chrom oder Inosit und bei dem Butyrat produzierenden Bakterium um *Intestinimonas butyriciproducens* oder Verwandte davon mit einer 16S-rRNA-Gensequenz mit einer Sequenzidentität von mindestens 97 % mit SEQ ID NO:3 oder *Anaerostipes rhamnosivorans* oder Verwandte davon mit einer 16S-rRNA-Gensequenz mit einer Sequenzidentität von mindestens 97 % mit SEQ ID NO:4 handelt,
wobei das mit Insulinresistenz verbundene Leiden aus der Gruppe bestehend aus metabolischem Syndrom, Diabetes mellitus Typ 1, Diabetes mellitus Typ 2, Dyslipidämie, Insulinresistenz bei endokriner Erkrankung, polyzystischem Ovarialsyndrom (PCOS), nichtalkoholischer Fettlebererkrankung (NAFLD) und/oder nichtalkoholischer Steatohepatitis (NASH) ausgewählt ist.

2. Insulinsensibilisierungsmittel in Kombination mit einem Butyrat produzierenden Bakterium zur Verwendung nach Anspruch 1, wobei das Butyrat produzierende Bakterium mikroverkapselt ist.

3. Insulinsensibilisierungsmittel in Kombination mit einem Butyrat produzierenden Bakterium zur Verwendung nach einem der vorhergehenden Ansprüche, wobei es sich bei dem Insulinsensibilisierungsmittel um Chrom und bei dem Butyrat produzierenden Bakterium um *Anaerobutyricum soehngenii* oder Verwandte davon mit einer 16S-rRNA-Gensequenz mit einer Sequenzidentität von mindestens 97 % mit SEQ ID NO:1 oder SEQ ID NO:2 handelt.

4. Insulinsensibilisierungsmittel in Kombination mit einem Butyrat produzierenden Bakterium zur Verwendung nach einem der vorhergehenden Ansprüche, wobei es sich bei dem Chrom um Chrom(III)picolinat handelt.

5. Insulinsensibilisierungsmittel in Kombination mit einem Butyrat produzierenden Bakterium zur Verwendung nach einem der vorhergehenden Ansprüche, wobei das Butyrat produzierende Bakterium getrennt von, nacheinander oder gleichzeitig mit dem Insulinsensibilisierungsmittel verabreicht wird.

6. Insulinsensibilisierungsmittel in Kombination mit einem Butyrat produzierenden Bakterium zur Verwendung nach einem der vorhergehenden Ansprüche, wobei das Butyrat produzierende Bakterium in einer Menge im Bereich von 10⁴ bis 10¹⁵ koloniebildenden Einheiten (Colony Forming Units, CFU) vorliegt.

7. Insulinsensibilisierungsmittel in Kombination mit einem Butyrat produzierenden Bakterium zur Verwendung nach einem der vorhergehenden Ansprüche, wobei das Butyrat produzierende Bakterium in lyophilisierter Form vorliegt.

8. Insulinsensibilisierungsmittel in Kombination mit einem Butyrat produzierenden Bakterium zur Verwendung nach einem der vorhergehenden Ansprüche, wobei Metformin in einer Menge im Bereich von 100 mg bis 5000 mg vorliegt.

9. Insulinsensibilisierungsmittel in Kombination mit einem Butyrat produzierenden Bakterium zur Verwendung nach einem der vorhergehenden Ansprüche, wobei Chrom in einer Menge im Bereich von 10 µg bis 5000 µg vorliegt.

10. Insulinsensibilisierungsmittel in Kombination mit einem Butyrat produzierenden Bakterium zur Verwendung nach einem der vorhergehenden Ansprüche, wobei Inosit in einer Menge im Bereich von 10 mg bis 100 g vorliegt.

11. Insulinsensibilisierungsmittel in Kombination mit einem Butyrat produzierenden Bakterium zur Verwendung nach einem der vorhergehenden Ansprüche, wobei das Insulinsensibilisierungsmittel und das Butyrat produzierende Bakterium in einer Zusammensetzung enthalten sind.

12. Insulinsensibilisierungsmittel in Kombination mit einem Butyrat produzierenden Bakterium zur Verwendung nach Anspruch 11, wobei die Zusammensetzung einen physiologisch unbedenklichen Träger umfasst.

13. Insulinsensibilisierungsmittel in Kombination mit einem Butyrat produzierenden Bakterium zur Verwendung nach einem der Ansprüche 11-12, wobei es sich bei der Zusammensetzung um eine pharmazeutische Zusammensetzung, vorzugsweise in einer fester Darreichungsform wie einer Kapsel, einer Tablette oder einem Pulver, handelt.

14. Insulinsensibilisierungsmittel in Kombination mit einem Butyrat produzierenden Bakterium zur Verwendung nach einem der Ansprüche 11-12, wobei es sich bei der Zusammensetzung um eine Lebensmittelzusammensetzung, bevorzugt ein Milchprodukt, bevorzugter ein fermentiertes Milchprodukt, am meisten bevorzugt einen Joghurt oder ein Joghurtgetränk handelt.

## Revendications

1. Agent insulinosensibilisant choisi dans le groupe constitué par
- chrome ;
- inositol ;
- metformine ; et
- sorbitol pour une utilisation dans la prévention et/ou le traitement de la résistance à l'insuline et/ou d'un état lié à la résistance à l'insuline, dans lequel l'agent insulinosensibilisant est combiné avec une bactérie productrice de butyrate choisie dans le groupe constitué par
- *Anaerobutyricum soehngenii* ou un parent de celle-ci ayant une séquence de gène d'ARNr 16S ayant au moins 97 % d'identité de séquence avec SEQ ID NO:1 ou SEQ ID NO:2 ;
- *Intestinimonas butyriciproducens* ou un parent de celle-ci ayant une séquence de gène d'ARNr 16S ayant au moins 97 % d'identité de séquence avec SEQ ID NO:3 ; et
- *Anaerostipes rhamnosivorans* ou un parent de celle-ci ayant une séquence de gène d'ARNr 16S ayant au moins 97 % d'identité de séquence avec SEQ ID NO:4,
dans lequel la bactérie productrice de butyrate est micro-encapsulée au cas où
- l'agent insulinosensibilisant est la metformine et la bactérie productrice de butyrate est *Anaerobutyricum soehngenii* ou un parent de celle-ci ayant une séquence de gène d'ARNr 16S ayant au moins 97 % d'identité de séquence avec SEQ ID NO:1 ou SEQ ID NO:2 ; ou
- l'agent insulinosensibilisant est le chrome ou l'inositol et la bactérie productrice de butyrate est *Intestinimonas butyriciproducens* ou un parent de celle-ci ayant une séquence de gène d'ARNr 16S ayant au moins 97 % d'identité de séquence avec SEQ ID NO:3 ou *Anaerostipes rhamnosivorans* ou un parent de celle-ci ayant une séquence de gène d'ARNr 16S ayant au moins 97 % d'identité de séquence avec SEQ ID NO:4,
dans lequel l'état lié à la résistance à l'insuline est choisi dans le groupe constitué par le syndrome métabolique, le diabète sucré de type 1, le diabète sucré de type 2, la dyslipidémie, la résistance à l'insuline dans une maladie endocrinienne, le syndrome des ovaires polykystiques (PCOS), la stéatohépatite non alcoolique (NAFLD), et/ou la stéatohépatite non alcoolique (NASH).

2. Agent insulinosensibilisant en combinaison avec une bactérie productrice de butyrate pour utilisation selon la revendication 1, dans lequel la bactérie productrice de butyrate est micro-encapsulée.

3. Agent insulinosensibilisant en combinaison avec une bactérie productrice de butyrate pour utilisation selon l'une quelconque des revendications précédentes, dans lequel l'agent insulinosensibilisant est le chrome, et la bactérie productrice de butyrate est Anaerobutyricum soehngenii ou un parent de celle-ci ayant une séquence de gène d'ARNr 16S ayant au moins 97 % d'identité de séquence avec SEQ ID NO:1 ou SEQ ID NO:2.

4. Agent insulinosensibilisant en combinaison avec une bactérie productrice de butyrate pour utilisation selon l'une quelconque des revendications précédentes, dans lequel le chrome est le picolinate de chrome(III).

5. Agent insulinosensibilisant en combinaison avec une bactérie productrice de butyrate pour utilisation selon l'une quelconque des revendications précédentes, dans lequel la bactérie productrice de butyrate est administrée séparément, séquentiellement ou simultanément à l'agent insulinosensibilisant.

6. Agent insulinosensibilisant en combinaison avec une bactérie productrice de butyrate pour utilisation selon l'une quelconque des revendications précédentes, dans lequel la bactérie productrice de butyrate est présente en une quantité allant de 10⁴ à 10¹⁵ unités formant colonie (UFC).

7. Agent insulinosensibilisant en combinaison avec une bactérie productrice de butyrate pour utilisation selon l'une quelconque des revendications précédentes, dans lequel la bactérie productrice de butyrate est présente sous forme lyophilisée.

8. Agent insulinosensibilisant en combinaison avec une bactérie productrice de butyrate pour utilisation selon l'une quelconque des revendications précédentes, dans lequel la metformine est présente en une quantité allant de 100 mg à 5 000 mg

9. Agent insulinosensibilisant en combinaison avec une bactérie productrice de butyrate pour utilisation selon l'une quelconque des revendications précédentes, dans lequel le chrome est présent en une quantité allant de 10 µg à 5 000 µg.

10. Agent insulinosensibilisant en combinaison avec une bactérie productrice de butyrate pour utilisation selon l'une quelconque des revendications précédentes, dans lequel l'inositol est présent en une quantité allant de 10 mg à 100 g.

11. Agent insulinosensibilisant en combinaison avec une bactérie productrice de butyrate pour utilisation selon l'une quelconque des revendications précédentes, dans lequel l'agent insulinosensibilisant et la bactérie productrice de butyrate sont compris dans une composition.

12. Agent insulinosensibilisant en combinaison avec une bactérie productrice de butyrate pour utilisation selon la revendication 11, dans lequel la composition comprend un support physiologiquement acceptable.

13. Agent insulinosensibilisant en combinaison avec une bactérie productrice de butyrate pour utilisation selon l'une quelconque des revendications 11-12, dans lequel la composition est une composition pharmaceutique, de préférence sous forme de dosage solide, telle qu'une capsule, un comprimé ou une poudre.

14. Agent insulinosensibilisant en combinaison avec une bactérie productrice de butyrate pour utilisation selon l'une quelconque des revendications 11-12, dans lequel la composition est une composition alimentaire, de préférence un produit laitier, plus préférablement un produit laitier fermenté, le plus préférablement un yaourt ou une boisson au yaourt.
